# EUROPÄISCHE PATENTSCHRIFT

(11) **EP 1 682 478 B1**
(45) Veröffentlichungstag und Bekanntmachung des Hinweises auf die Patenterteilung: **11.05.2016**
(21) Anmeldenummer: 04790946.0
(22) Anmeldetag: 28.10.2004
(51) Int. Cl.: C07C 51/25, C07C 57/04

(54) **VERFAHREN ZUM LANGZEITBETRIEB EINER HETEROGEN KATALYSIERTEN GASPHASENPARTIALOXIDATION VON PROPEN ZU ACRYLSÄURE**
LONG-LIFE METHOD FOR HETEROGENEOUSLY-CATALYSED GAS PHASE PARTIAL OXIDATION OF PROPENE INTO ACRYLIC ACID
PROCEDE LONGUE DUREE D'OXYDATION PARTIELLE EN PHASE GAZEUSE A CATALYSE HETEROGENE DE PROPENE EN ACIDE ACRYLIQUE

(30) Priorität: 31.10.2003 US 515659 P; 31.10.2003 DE 10351269
(43) Veröffentlichungstag der Anmeldung: 26.07.2006
(73) Patentinhaber: BASF SE, 67056 Ludwigshafen am Rhein (DE)
(72) Erfinder: DIETERLE, Martin, 68167 Mannheim (DE); PETZOLDT, Jochen, 67273 Weisenheim am Berg (DE); MÜLLER-ENGEL, Klaus Joachim, 76297 Stutensee (DE)
(74) Vertreter: BASF IP Association
(86) Internationale Anmeldenummer: PCT/EP2004/012173
(87) Internationale Veröffentlichungsnummer: WO 2005/042459

(56) Entgegenhaltungen:
- EP-A- 0 614 872
- US-B1- 6 346 646

## Beschreibung

Vorliegende Erfindung betrifft ein Verfahren zum Langzeitbetrieb einer heterogen katalysierten Gasphasenpartialoxidation von Propen zu Acrylsäure, bei dem man ein Propen, molekularen Sauerstoff und wenigstens ein Inertgas enthaltendes Reaktionsgasausgangsgemisch 1, das den molekularen Sauerstoff und das Propen in einem molaren Verhältnis O₂:C₃H₆≥ 1 enthält, zunächst in einer ersten Reaktionsstufe bei erhöhter Temperatur so über ein erstes Katalysatorfestbett 1, dessen Katalysatoren so beschaffen sind, dass ihre Aktivmasse wenigstens ein Molybdän und/oder Wolfram sowie wenigstens eines der Elemente Wismut, Tellur, Antimon, Zinn und Kupfer enthaltendes Multimetalloxid ist, führt, dass der Propenumsatz bei einmaligem Durchgang ≥ 93 mol.-% und die damit einhergehende Selektivität der Acroleinbildung sowie der Acrylsäurenebenproduktbildung zusammengenommen ≥ 90 mol-% betragen, die Temperatur des die erste Reaktionsstufe verlassenden Produktgasgemischs 1 durch direkte und/oder indirekte Kühlung gegebenenfalls verringert und dem Produktgasgemisch 1 gegebenenfalls molekularen Sauerstoff und/oder Inertgas zugibt, und danach das Produktgasgemisch 1 als Acrolein, molekularen Sauerstoff und wenigstens ein Inertgas enthaltendes Reaktionsgasausgangsgemisch 2, das den molekularen Sauerstoff und das Acrolein in einem molaren Verhältnis O₂:C₃H₄O ≥ 0,5 enthält, in einer zweiten Reaktionsstufe bei erhöhter Temperatur so über ein zweites Katalysatorfestbett 2, dessen Katalysatoren so beschaffen sind, dass ihre Aktivmasse wenigstens ein die Elemente Molybdän und Vanadin enthaltendes Multimetalloxid ist, führt, dass der Acroleinumsatz bei einmaligen Durchgang ≥ 90 mol-% und die Selektivität der über beide Reaktionsstufen bilanzierten Acrylsäurebildung, bezogen auf umgesetztes Propen, ≥ 80 mol-% beträgt, und bei dem man, um der Deaktivierung sowohl des Katalysatorfestbetts 1 als auch des Katalysatorfestbetts 2 entgegenzuwirken, die Temperatur des jeweiligen Katalysatorfestbetts unabhängig voneinander über die Zeit erhöht.

Acrylsäure ist ein bedeutendes Monomer, das als solches oder in Form seiner Alkyl-ester zur Erzeugung von z.B. als Klebstoffen geeigneten Polymerisaten Verwendung findet.

Es ist bekannt, Acrylsäure durch zweistufige heterogen katalysierte Gasphasenpartialoxidation von Propen zu Acrylsäure im Katalysatorfestbett herzustellen (vgl. z.B. EP-A 1159247, DE-A 10313208, DE-A 19948248, EP-A 990636, EP-A 1106598, DE-A 3002829). In der ersten Reaktionsstufe wird Propen im wesentlichen partiell zu Acrolein oxidiert und in der zweiten Reaktionsstufe wird das in der ersten Reaktionsstufe gebildete Acrolein im wesentlichen partiell zu Acrylsäure oxidiert. Von Bedeutung ist dabei, dass die technische Ausführungsform normalerweise so ausgestaltet ist, dass das in der ersten Reaktionsstufe gebildete Acrolein nicht abgetrennt, sondern als Bestandteil des die erste Reaktionsstufe verlassenden Produktgasgemischs, gegebenenfalls durch molekularen Sauerstoff und Inertgas ergänzt, und gegebenenfalls durch direkte und/oder indirekte Kühlung abgekühlt, in die zweite Reaktionsstufe geführt wird. Das in der jeweiligen Reaktionsstufe verwendete Katalysatorfestbett ist für die jeweilige Reaktionsstufe maßgeschneidert und von dem für die andere Reaktionsstufe verwendeten Katalysatorfestbett verschieden. Außerdem erfolgen die Temperierungen der beiden Reaktionsstufen unabhängig voneinander so, dass sich das jeweilige Katalysatorfestbett auf seiner optimalen Arbeitstemperatur befindet.

Im besonderen sind die beiden Reaktionsstufen für sich bekannt (vgl. z.B. EP-A 714 700, EP-A 700 893, EP-A 279 374, EP-A 575 897 etc.).

Es ist auch bekannt, dass ein solches zweistufiges Verfahren der heterogen katalysierten Gasphasenpartialoxidation von Propen zu Acrylsäure an ein und denselben Katalysatorfestbetten im wesentlichen kontinuierlich über längere Zeiträume betrieben werden kann. Allerdings verlieren die Katalysatorfestbetten dabei im Verlauf der Betriebszeit an Qualität. In der Regel verschlechtern sich in beiden Stufen sowohl ihre Aktivität als auch die Selektivität der jeweiligen Zielproduktbildung.

Um die Katalysatorfestbetten, deren Fertigung und Austausch vergleichsweise aufwendig und kostspielig sind, trotzdem möglichst lange in einem damit beschickten Reaktorsystem betreiben zu können, wird im Stand der Technik auf unterschiedlichste Art und Weise versucht, ihrem Alterungsprozess entgegenzuwirken.

Die EP-A 990 636 (z.B. Seite 8, Zeilen 13 bis 15), die EP-A 1070700 (z.B. Seite 4, Zeilen 49, 50) und die EP-A 1 106 598 (z.B. Seite 13, Zeilen 43 bis 45) schlagen vor, die Minderung der Qualität des jeweiligen Katalysatorfestbetts dadurch weitgehend zu kompensieren, dass im Verlauf der Betriebszeit, unter ansonsten weitgehend gleichbleibenden Betriebsbedingungen, die Temperatur des jeweiligen Katalysatorfestbetts nach und nach erhöht wird, um den Propen- bzw. Acroleinumsatz bei einmaligem Durchgang des Reaktionsgasgemischs durch die Katalysatorfestbetten im wesentlichen beizubehalten.

Unter der Temperatur des Katalysatorfestbetts wird dabei die Temperatur des Katalysatorfestbetts bei Ausübung des Partialoxidationsverfahrens jedoch in fiktiver Abwesenheit einer chemischen Reaktion (d.h. ohne den Einfluss der Reaktionswärme) verstanden. Dies soll auch in dieser Schrift gelten. Unter effektiver Temperatur des jeweiligen Katalysatorfestbetts soll in dieser Schrift dagegen die tatsächliche Temperatur des Katalysatorfestbetts unter Einbezug der Reaktionswärme der Partialoxidation verstanden werden. Ist die Temperatur eines Katalysatorfestbetts längs des Katalysatorfestbetts nicht konstant (z.B. im Fall von mehreren Temperaturzonen), so meint der Begriff Temperatur des Katalysatorfestbetts in dieser Schrift den (Zahlen) Mittelwert der Temperatur entlang des Katalysatorfestbetts.

Von Bedeutung im vorgenannten Zusammenhang ist, dass die Temperatur des Reaktionsgasgemischs (und damit auch die effektive Temperatur eines Katalysatorfestbetts) beim Durchgang durch ein Katalysatorfestbett einen Höchstwert (den sogenannten Heißpunktwert) durchläuft. Die Differenz zwischen Heißpunktwert und der Temperatur des Katalysatorfestbetts an der Stelle des Heißpunktwertes wird als Heißpunktausdehnung bezeichnet.

Nachteilig an der in der EP-A 990 636 sowie in der EP-A 1 106 598 empfohlenen Verfahrensweise ist, dass sich mit zunehmender Erhöhung der Temperatur des Katalysatorfestbetts sein Alterungsprozess beschleunigt (bestimmte Bewegungsprozesse innerhalb der Aktivmassen der Katalysatoren, die zur Alterung beitragen, laufen z.B. schneller ab). Dies in der Regel vor allem auch deshalb, weil die Heißpunktausdehnung mit einer Erhöhung der Temperatur des Katalysatorfestbetts meist noch stärker zunimmt als die Temperatur des Katalysatorfestbetts selbst (vgl. z.B. Seite 12, Zeilen 45 bis 48 der EP-A 1 106 598 und Seite 8, Zeilen 11 bis 15 der EP-A 990 636). Die effektive Temperatur des Katalysatorfestbetts nimmt im Heißpunktbereich daher meist überproportional zu, was die Alterung des Katalysatorfestbetts zusätzlich fördert.

Bei Erreichen eines Höchstwerts der Temperatur des Katalysatorfestbetts wird daher das Katalysatorfestbett üblicherweise vollständig ausgetauscht.

Nachteilig an einem solchen Komplettaustausch ist jedoch, dass er vergleichsweise aufwendig ist. Das Verfahren der Acrylsäureherstellung muss für längere Zeit unterbrochen werden und die Kosten der Katalysatorherstellung sind ebenfalls erheblich.

Erwünscht sind daher Betriebsweisen für Verfahren einer zweistufigen heterogen katalysierten Gasphasenpartialoxidation von Propen zu Acrylsäure, die dabei behilflich sind, die Standzeit der Katalysatorfestbetten im Reaktorsystem weitergehend in die Länge zu ziehen.

Die DE-A 102 32 748 empfiehlt diesbezüglich, anstatt das Katalysatorfestbett vollständig auszutauschen, nur eine Teilmenge desselben durch eine frische Katalysatorbeschickung zu ersetzen.

Nachteilig an diesem Vorschlag ist, dass auch mit einem Teilwechsel des Katalysatorfestbetts bereits ein nennenswerter Aufwand einhergeht.

Die EP-A 614 872, die insbesondere die zweite Reaktionsstufe, in der das Acrolein partiell zur Acrylsäure oxidiert wird, betrifft, empfiehlt, die Standzeit eines Zweitstufenkatalysatorfestbetts dadurch zu verlängern, dass man nach mehrjähriger Betriebsdauer des Katalysatorfestbetts, mit der Erhöhungen der Temperatur desselben von 15°C bis 30°C und mehr einhergehen, das Verfahren der Partialoxidation unterbricht, und bei Katalysatorfestbetttemperaturen von 260 bis 450°C durch selbiges ein Gasgemisch aus Sauerstoff, Wasserdampf und Inertgas führt und anschließend die Partialoxidation fortsetzt.

In diesem Zusammenhang sollen in dieser Schrift als Inertgase in einem Gasgemisch, das unter bestimmten Bedingungen durch ein Katalysatorfestbett geführt wird, solche Gase verstanden werden, die bei der Durchführung durch das Katalysatorfestbett zu wenigstens 95 mol-%, bevorzugt zu wenigstens 98 mol-%, ganz besonders bevorzugt zu wenigstens 99 mol-% oder 99,5 mol-% unverändert erhalten bleiben. Das erfindungsgemäß einzusetzende Gasgemisch G betreffend soll Wasserdampf nicht unter dem Begriff Inertgas subsummieren.

Die EP-A 169 449, die im wesentlichen die erste Reaktionsstufe, in der das Propen partiell zu Acrolein oxidiert wird, betrifft, empfiehlt, die Standzeit des Katalysatorfestbetts dadurch zu verlängern, dass man nach mehrjähriger Betriebsdauer des Katalysatorfestbetts, mit der Erhöhungen der Temperatur desselben von 15°C und mehr einhergehen, das Verfahren der Partialoxidation unterbricht, und bei Katalysatorfestbetttemperaturen von 380 bis 540°C durch selbiges ein im wesentlichen aus Luft bestehendes Gas führt und anschließend die Partialoxidation fortsetzt.

Die EP-A 339119 empfiehlt bei analoger Verfahrensweise die Anwendung eines Sauerstoff und Wasserdampf enthaltenden Gases.

Nachteilig an den Verfahrensweisen der EP-A 339119, der EP-A 169449 sowie der EP-A 614872 ist jedoch, das bis zum Zeitpunkt der Unterbrechung der Partialoxidation die Alterung des Katalysatorfestbetts ungebremst fortschreitet und gefördert wird.

Wünschenswert wäre daher insbesondere ein Verfahren zum Langzeitbetrieb einer zweistufigen heterogen katalysierten Gasphasenpartialoxidation von Propen zu Acrylsäure, bei dem der Katalysatoralterung in beiden Stufen auf eine Art und Weise entgegengewirkt wird, mit der die Ausprägung der Heißpunktausdehnung in beiden Stufen über die Zeit geringer ist als bei den Verfahren des Standes der Technik.

Demgemäss wurde ein Verfahren zum Langzeitbetrieb einer heterogen katalysierten Gasphasenpartialoxidation von Propen zu Acrylsäure, bei dem man ein Propen, molekularen Sauerstoff und wenigstens ein Inertgas enthaltendes Reaktionsgasausgangsgemisch 1, das den molekularen Sauerstoff und das Propen in einem molaren Verhältnis O₂:C₃H₆≥ 1 enthält, zunächst in einer ersten Reaktionsstufe bei erhöhter Temperatur so über ein erstes Katalysatorfestbett 1, dessen Katalysatoren so beschaffen sind, dass ihre Aktivmasse wenigstens ein Molybdän und/oder Wolfram sowie wenigstens eines der Elemente Wismut, Tellur, Antimon, Zinn und Kupfer enthaltendes Multimetalloxid ist, führt, dass der Propenumsatz bei einmaligem Durchgang ≥ 93 mol-% und die damit einhergehende Selektivität der Acroleinbildung sowie der Acrylsäurenebenproduktbildung zusammengenommen ≥ 90 mol-% (vorzugsweise ≥ 92 mol-%, bzw. ≥ 94 mol-%, bzw. ≥ 96 mol-%, bzw. ≥ 98 mol-%) betragen, die Temperatur des die erste Reaktionsstufe verlassenden Produktgasgemischs 1 durch direkte und/oder indirekte Kühlung gegebenenfalls verringert und dem Produktgasgemisch 1 gegebenenfalls molekularen Sauerstoff und/oder Inertgas zugibt, und danach das Produktgasgemisch 1 als Acrolein, molekularen Sauerstoff und wenigstens ein Inertgas enthaltendes Reaktionsgasausgangsgemisch 2, das den molekularen Sauerstoff und das Acrolein in einem molaren Verhältnis O₂:C₃H₄O≥ 0,5 enthält, in einer zweiten Reaktionsstufe bei erhöhter Temperatur so über ein zweites Katalysatorfestbett 2, dessen Katalysatoren so beschaffen sind, dass ihre Aktivmasse wenigstens ein die Elemente Molybdän und Vanadin enthaltendes Multimetalloxid ist, führt, dass der Acroleinumsatz bei einmaligem Durchgang ≥ 90 mol-% und die Selektivität der über beide Reaktionsstufen bilanzierten Acrylsäurebildung, bezogen auf umgesetztes Propen, ≥ 80 mol-% (vorzugsweise ≥ 83 mol-%, bzw. ≥ 85 mol-%, bzw. ≥ 88 mol-%, bzw. ≥ 90 mol-%, bzw. ≥ 93 mol-%) beträgt, und bei dem man, um der Deaktivierung sowohl des Katalysatorfestbetts 1 als auch des Katalysatorfestbetts 2 entgegenzuwirken, die Temperatur des jeweiligen Katalysatorfestbetts unabhängig voneinander über die Zeit erhöht, gefunden, das dadurch gekennzeichnet ist, dass man die Gasphasenpartialoxidation wenigstens einmal innerhalb von 7500 Betriebsstunden, bevorzugt wenigstens einmal innerhalb von 4000 Betriebsstunden, unterbricht und bei einer Temperatur des Katalysatorfestbetts 1 von 250 bis 550°C und einer Temperatur des Katalysatorfestbetts 2 von 200 bis 450°C ein aus molekularem Sauerstoff, Inertgas und gegebenenfalls Wasserdampf bestehendes Gasgemisch G zunächst durch das Katalysatorfestbett 1, daran anschließend gegebenenfalls durch einen Zwischenkühler und abschließend durch das Katalysatorfestbett 2 führt.

Es überrascht, dass bei Anwendung des erfindungsgemäßen Verfahrens ein Langzeitbetrieb einer zweitstufigen heterogen katalysierten Gasphasenpartialoxidation von Propen zu Acrylsäure möglich ist, mit dem die Ausprägung der Heißpunktausdehnungen über die Zeit geringer ist als bei den Verfahren des Standes der Technik. In günstigen Fällen bleiben die Ausprägungen der Heißpunktausdehnung über die Zeit konstant oder verhalten sich sogar abnehmend. Außerdem bleibt die Selektivität der jeweiligen Zielproduktbildung über die Zeit meist weitgehend konstant und nimmt in günstigen Fällen sogar zu.

So unterbricht man die Gasphasenpartialoxidation bevorzugt wenigstens einmal, bevor die Temperaturerhöhung des Katalysatorfestbetts 2 dauerhaft ≥ 10°C beträgt, besonders bevorzugt bevor die Temperaturerhöhung des Katalysatorfestbetts 2 dauerhaft ≥ 8°C, oder ≥ 7°C, oder ≥ 6°C, oder ≥ 5°C, oder ≥ 4°C beträgt, um in erfindungsgemäßer Weise das Gasgemisch G durch die Katalysatorfestbetten zu führen.

Besonders bevorzugt wird man beim erfindungsgemäßen Verfahren bereits bevor die Temperaturerhöhung des Katalysatorfestbetts 2 dauerhaft ≥ 3°C oder ≥ 2°C beträgt die Gasphasenpartialoxidation wenigstens einmal unterbrechen, um in erfindungsgemäßer Weise das Gasgemisch G durch die Katalysatorfestbetten zu führen.

Das erfindungsgemäße Verfahren ist aber auch dann vorteilhaft, wenn, bevor die Temperaturerhöhung des Katalysatorbetts 2 dauerhaft ≥ 1°C oder weniger beträgt, die Gasphasenpartialoxidation wenigstens einmal unterbrochen und in erfindungsgemäßer Weise das Gasgemisch G durch die Katalysatorfestbetten geführt wird.

In der Regel wird die Temperaturerhöhung des Katalysatorfestbetts 2 jedoch dauerhaft ≥ 0,1°C oder ≥ 0,2°C betragen, bevor die Gasphasenpartialoxidation von Propen zu Acrylsäure wenigstens einmal erfindungsgemäß unterbrochen wird.

Der Wortlaut "bevor die durchgeführte Temperaturerhöhung des Katalysatorfestbetts 2 dauerhaft ≥ 10°C oder ≥ 8°C (allgemein ≥ X°C) beträgt" berücksichtigt, dass die Temperatur eines Katalysatorfestbetts 2 im großtechnischen Betrieb aus unterschiedlichen Gründen gewissen Schwankungen unterliegen kann. In diesem Fall trägt man den tatsächlichen Verlauf der Temperatur des Katalysatorfestbetts 2 über die Zeit auf und legt durch die Messpunkte nach der von Legendre und Gauß entwickelten Methode der kleinsten Summe der Abweichungsquadrate eine Ausgleichskurve. Ist auf dieser Ausgleichskurve eine Temperaturerhöhung von ≥ 10°C oder ≥ 8°C (allgemein von ≥ X°C) erreicht, gilt das Merkmal "dauerhaft" als erfüllt.

Erfindungsgemäß vorteilhaft wird die Temperatur des Katalysatorfestbetts 1 beim erfindungsgemäßen Durchleiten von Gasgemisch G 300 bis 500°C, besonders bevorzugt 300 bis 400°C, und ganz besonders bevorzugt 300 bis 360°C betragen.

Die Temperatur des Katalysatorfestbetts 2 wird beim erfindungsgemäßen Durchleiten von Gasgemisch G vorzugsweise 250 bis 400°C, oft 250 bis 350°C und vielfach 250 bis 300°C betragen.

Erfindungsgemäß günstig wird man die Temperatur des Katalysatorfestbetts 1 während der Durchführung des Gasgemischs G bei der Durchführung des erfindungsgemäßen Verfahrens auf einem Wert T_{G1} halten, der im wesentlichen derjenigen Temperatur T_{V1} des Katalysatorfestbetts 1 entspricht, das dieses beim Betrieb der Partialoxidation aufwies, bevor diese unterbrochen wurde, um das Gasgemisch G erfindungsgemäß durch das Katalysatorfestbett 1 zu führen.

D.h., erfindungsgemäß vorteilhaft gilt T_{G1}=T_{V1}±50°C bzw. T_{G1}=T_{V1}±20°C und ganz besonders vorteilhaft ist T_{G1}=T_{V1}.

Normalerweise wird T_{V1} im Bereich von 250 bis 450°C, häufig im Bereich von 300 bis 400°C liegen.

Bevorzugt wird man die Temperatur des Katalysatorfestbetts 2 während der erfindungsgemäßen Durchführung des Gasgemischs G auf einem Wert T_{G2} halten, der im wesentlichen derjenigen Temperatur T_{V2} des Katalysatorfestbetts 2 entspricht, die das Katalysatorfestbett 2 während des Betriebs der Partialoxidation aufwies, bevor diese zum Zweck der erfindungsgemäßen Durchführung des Gasgemischs G unterbrochen wurde.

D.h., erfindungsgemäß vorteilhaft gilt T_{G2}=T_{V2}±50°C bzw. T_{G2}=T_{V2}±20°C und ganz besonders vorteilhaft ist T_{G2}=T_{V2}. Normalerweise wird T_{V2} im Bereich von 200 bis 400°C, häufig im Bereich von 220 bis 350°C liegen.

Die Zeitdauer t_{G} während der beim erfindungsgemäßen Verfahren das Gasgemisch G durch die Katalysatorbetten zu führen ist, wird in der Regel wenigstens 2h, häufig 6h bis 120h, vielfach 12h bis 72h und oft 20h bis 40h betragen. Sie kann jedoch auch 10 Tage und mehr betragen. In der Regel wird ein kleiner Sauerstoffgehalt des Gasgemischs G eine längere Zeitdauer t_{G} bedingen. Erhöhte Sauerstoffgehalte im Gasgemisch G sind erfindungsgemäß vorteilhaft.

Üblicherweise wird man die Zeitdauer t_{G} wenigstens so lange bemessen, bis der Sauerstoffgehalt des Gasgemischs G beim Austritt aus dem Katalysatorfestbett 2 sich vom Sauerstoffgehalt des Gasgemischs G beim Eintritt in das Katalysatorfestbett 1 nicht mehr unterscheidet.

Zwischen den Katalysatorfestbetten 1 und 2 kann beim erfindungsgemäßen Verfahren gegebenenfalls mit frischem Gasgemisch G ergänzt werden. Bevorzugt wird beim erfindungsgemäßen Verfahren eine solche Ergänzung jedoch nicht vorgenommen.

In der Regel wird man beim erfindungsgemäßen Verfahren die erfindungsgemäße Durchführung des Gasgemischs G durch die Katalysatorfestbetten in einer Häufigkeiten H von wenigstens einmal pro Kalenderjahr, vorzugsweise von wenigstens einmal pro Kalenderdreivierteljahr bzw. pro Kalenderhalbjahr, besonders bevorzugt von wenigstens einmal pro Kalenderquartal und besonders bevorzugt von wenigstens einmal pro Kalendermonat durchführen.

In anderen Worten ausgedrückt wird man beim erfindungsgemäßen Verfahren die erfindungsgemäße Durchführung des Gasgemischs G durch die Katalysatorfestbetten wenigstens einmal innerhalb von 7500 oder 7000, oder 6000, bevorzugt wenigstens einmal innerhalb von 5500 oder 5000 und ganz besonders bevorzugt wenigstens einmal innerhalb von 4000, oder 3000, oder 2000, oder 1500, oder 1000, oder 500 Betriebsstunden der Partialoxidation durchführen. Eine häufige Durchführung des erfindungsgemäßen Verfahrens wirkt sich vorteilhaft aus.

Im übrigen wird man das Verfahren der heterogen katalysierten Gasphasenpartialoxidation von Propen zu Acrylsäure weitgehend kontinuierlich durchführen.

Häufig wird man beim erfindungsgemäßen Verfahren die Erhöhung der Temperatur des Katalysatorfestbetts 1 so durchführen, dass der Propenumsatz bei einmaligem Durchgang des Reaktionsgasgemischs durch das Katalysatorfestbett 1 93 mol-%, bzw. 94 mol-%, bzw. 95 mol-%, bzw. 96 mol-%, bzw. 97 mol-% nicht unterschreitet.

D.h., normalerweise wird man die Temperatur des Katalysatorfestbetts 1 wenigstens einmal erhöhen bevor 7500 oder 7000, meist bevor 6000, und vielfach bevor 5000, oder 4000, oder 3000 Betriebsstunden der Partialoxidation erreicht sind.

In besonders zweckmäßiger Weise wird man die Erhöhung der Temperatur des Katalysatorfestbetts 1 beim erfindungsgemäßen Verfahren unter Anwendung besonders günstiger Katalysatoren (z.B. den in dieser Schrift empfohlenen) bevorzugt (meist im wesentlichen kontinuierlich und) so durchführen, dass der Propengehalt im Produktgasgemisch der ersten Reaktionsstufe den Wert 10000 Gew.-ppm, vorzugsweise 6000 Gew.-ppm und besonders bevorzugt 4000 bzw. 2000 Gew.-ppm nicht übersteigt.

Ferner sollte der Restsauerstoff im Produktgasgemisch der ersten Reaktionsstufe wenigstens 1 Vol.-%, vorzugsweise wenigstens 2 Vol.-% und besonders bevorzugt wenigstens 3 Vol.-% betragen.

Die Erhöhung der Temperatur des Katalysatorfestbetts 2 wird man beim erfindungsgemäßen Verfahren in ähnlicher Weise zweckmäßig so durchführen, dass der Acroleinumsatz bei einmaligem Durchgang des Reaktionsgasgemischs durch das Katalysatorfestbett 2 90 mol-%, bzw. 92 mol-%, bzw. 93 mol-% oder 94 mol-%, bzw. 96 mol-%, bzw. 98 mol-%, bzw. 99 mol-% nicht unterschreitet.

D.h., normalerweise wird man die Temperatur des Katalysatorfestbetts 2 wenigstens einmal erhöhen, bevor 7500 oder bevor 7000, meist bevor 6000, und vielfach bevor 5000, oder 4000, oder 3000 Betriebsstunden der Partialoxidation erreicht sind.

In besonders zweckmäßiger Weise wird man die Erhöhung der Temperatur des Katalysatorfestbetts 2 beim erfindungsgemäßen Verfahren unter Anwendung besonders günstiger Katalysatoren (z.B. der in dieser Schrift oder der in der JP-A 7-10802 empfohlenen) bevorzugt (meist im wesentlichen kontinuierlich und) so durchführen, dass der Acroleingehalt im Produktgasgemisch der ersten Reaktionsstufe den Wert 1500 Gew.-ppm, vorzugsweise 600 Gew.-ppm und besonders bevorzugt 350 Gew.-ppm nicht übersteigt. Dabei wird berücksichtigt, dass Acrolein bei den Verfahren der Abtrennung von Acrylsäure aus dem Produktgasgemisch der Partialoxidation insofern störend wirkt, als es die Polymerisationsneigung von Acrylsäure fördert (vgl. EP-A 1 041 062).

Ferner sollte der Restsauerstoff im Produktgasgemisch der zweiten Reaktionsstufe wenigstens 1 Vol.-%, vorzugsweise wenigstens 2 Vol.-% und besonders bevorzugt wenigstens 3 Vol.-% betragen.

In anwendungstechnisch zweckmäßiger Weise wird das Gasgemisch G beim erfindungsgemäßen Verfahren wenigstens 1 Vol.-% bzw. 2 Vol.-%, vorzugsweise wenigstens 3 Vol.-% und besonders bevorzugt wenigstens 4 Vol.-% Sauerstoff enthalten. In der Regel wird der Sauerstoffgehalt des Gasgemischs G jedoch ≤ 21 Vol.-% betragen. D.h., ein mögliches Gasgemisch G ist Luft. Ein anderes mögliches Gasgemisch G ist Magerluft. Dabei handelt es sich um an Sauerstoff entreicherte Luft.

Erfindungsgemäß vorteilhaft ist Magerluft, die aus 3 bis 10, vorzugsweise 4 bis 6 Vol.-% Sauerstoff und als Restmenge aus molekularem Stickstoff besteht. Erfindungsgemäß zweckmäßig enthält das Gasgemisch G im wesentlichen keinen Wasserdampf. Das erfindungsgemäß zu verwendende Gasgemisch G kann jedoch bis zu 0,1 Vol.-%, oder bis zu 0,5 Vol.-%, oder bis zur 1 Vol.-% an Wasserdampf enthalten. Normalerweise liegt der Wasserdampfgehalt des Gasgemischs G bei ≤ 75 Vol.-%. Der Inertgasanteil des Gasgemischs G liegt in der Regel bei ≤ 95 Vol.-%, meist bei ≤ 90 Vol.-%.

Erfindungsgemäß geeignete Gasgemische G können somit z.B. aus 3 bis 20 Vol.-%, vorzugsweise 4 bis 6 Vol.-% Sauerstoff, 0 bis 5 Vol.-% Wasserdampf und als Restmenge aus Inertgas bestehen. Bevorzugte Inertgase sind N₂ und CO₂. Insbesondere kommen für das erfindungsgemäße Verfahren alle in der EP-A 339119 und in der EP-A 169 449 sowie in der EP-A 614 872 empfohlenen Gasgemische G in Betracht. Ebenfalls können für das erfindungsgemäße Verfahren alle in diesen Schriften empfohlenen Regenerierbedingungen angewendet werden.

Die Menge des beim erfindungsgemäßen Verfahren durch das Katalysatorfestbett 1 geführten Gasgemischs G kann 5 oder 100 bis 5000 NI/I•h, vorzugsweise 20 oder 200 bis 3000 NI/I•h betragen. Bezugsbasis ist dabei das Volumen des gesamten Katalysatorfestbetts 1, d.h., einschließlich gegebenenfalls mitverwendeter Abschnitte, die ausschließlich aus Inertmaterial bestehen. Hohe Belastungen mit Gasgemisch G haben sich erfindungsgemäß als vorteilhaft erwiesen.

Als für das erfindungsgemäße Verfahren geeignete Multimetalloxidmasse kommen für die Katalysatoren der ersten Reaktionsstufe insbesondere Mo, Bi und Fe enthaltende aktive Multimetalloxide in Betracht. Dies sind vor allem die in der DE-A 103 44 149 und in der DE-A 103 44 264 offenbarten Mo, Bi und Fe enthaltenden Multimetalloxidmassen.

Dies sind insbesondere auch die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formeln I, II und III der DE-A 10101695, die Multimetalloxidaktivmassen der allgemeinen Formeln I, II und III der DE-A 19948248 und die Multimetalloxidaktivmassen der allgemeinen Formeln I, II und III der DE-A 19955168 sowie die in der EP-A 700714 genannten Multimetalloxidaktivmassen.

Ferner eignen sich für das erfindungsgemäß zu verwendende Katalysatorfestbett 1 die Mo, Bi und Fe enthaltenden Multimetalloxidkatalysatoren die in den Schriften DE-A 10046957, DE-A 10063162, DE-C 3338380, DE-A 19902562, EP-A 15565, DE-C 2380765, EP-A 807465, EP-A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden. Dies gilt insbesondere für die beispielhaften Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1 c aus der EP-A 15565 sowie ein in entsprechender Weise herzustellender Katalysator, dessen Aktivmasse jedoch die Zusammensetzung Mo₁₂Ni_{6,5}Zn₂Fe₂Bi₁P_{0,0065}K_{0,06}Ox•10 SiO₂ aufweist. Ferner sind hervorzuheben das Beispiel mit der laufenden Nr. 3 aus der DE-A 19855913 (Stöchiometrie: Mo₁₂Co₇Fe₃Bi_{0,6}K_{0,08}Si_{1,6}Ox) als Hohlzylindervollkatalysator der Geometrie 5 mm x 3 mm x 2 mm bzw. 5 mm x 2 mm x 2 mm (jeweils Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese eine Hohlzylinder-Geometrie der Ausmaße 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Ebenso eignen sich die Multimetalloxidkatalysatoren und Geometrien der DE-A 10101695 bzw. WO 02/062737.

Weiterhin sind das Beispiel 1 aus der DE-A 10046957 (Stöchiometrie: [Bi₂W₂O₉ x 2WO₃]_{0,5}•[Mo₁₂Co_{5,6}Fe_{2,94}Si_{1,59}K_{0,08}Oₓ]₁) als Hohlzylinder (Ring) vollkatalysator der Geometrie 5 mm x 3 mm x 2 mm bzw. 5 mm x 2 mm x 2 mm (jeweils Außendurchmesser x Länge x Innendurchmesser), sowie die Schalenkatalysatoren 1, 2 und 3 aus der DE-A 10063162 (Stöchiometrie: Mo₁₂Bi_{1,0}Fe₃Co₇Si_{1,6}K_{0,08}), jedoch als ringförmige Schalenkatalysatoren entsprechender Schalendicke und auf Trägerringe der Geometrie 5 mm x 3 mm x 1,5 mm bzw. 7 mm x 3 mm x 1,5 mm (jeweils Außendurchmesser x Länge x Innendurchmesser) aufgebracht, geeignet.

Eine Vielzahl der für die Katalysatoren des für das erfindungsgemäße Verfahren erforderlichen Katalysatorfestbetts 1 geeigneten Multimetalloxidaktivmassen lässt sich unter der allgemeinen Formel I,

Mo₁₂BiₐFe_{b}X¹_{c}X²_{d}X³ₑX⁴_{f}Oₙ (I),

in der die Variablen nachfolgende Bedeutung aufweisen:
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,
a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,
subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel I in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen aus der Sprühturm von 100 bis 150°C erfolgt.

Üblicherweise werden die Multimetalloxidaktivmassen der allgemeinen Formel I im beim erfindungsgemäßen Verfahren erforderlichen Katalysatorfestbett 1 nicht in Pulverform sondern zu bestimmten Katalysatorgeometrien geformt eingesetzt, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren in der ersten Reaktionsstufe unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm bzw. 5 mm x 3 mm x 2 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für die Katalysatoren des Katalysatorfestbetts 1 des erfindungsgemäßen Verfahrens geeignete Multimetalloxidaktivmassen sind ferner Massen der allgemeinen Formel II,

[Y¹_{a'}Y²_{b'}O_{x'}]ₚ[Y³_{c'}Y⁴_{d'}Y⁵_{e'}Y⁶_{f'}Y⁷_{g'}Y²_{h'}O_{y'}]_{q} (II),

in der die Variablen folgende Bedeutung haben:
- Y¹ =: nur Wismut oder Wismut und wenigstens eines der Elemente Tellur, Antimon, Zinn und Kupfer,
- Y² =: Molybdän oder Molybdän und Wolfram,
- Y³ =: ein Alkalimetall, Thallium und/oder Samarium,
- Y⁴ =: ein Erdalkalimetall, Nickel, Kobalt, Kupfer, Mangan, Zink, Zinn, Cadmium und/oder Quecksilber,
- Y⁵ =: Eisen oder Eisen und wenigstens eines der Elemente Chrom und Cer,
- Y⁶ =: Phosphor, Arsen, Bor und/oder Antimon,
- Y⁷ =: ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

- a' =: 0,01 bis 8,
- b' =: 0,1 bis 30,
- c' =: 0 bis 4,
- d' =: 0 bis 20,
- e' >: 0 bis 20,
- f' =: 0 bis 6,
- g' =: 0 bis 15,
- h' =: 8 bis 16,
- x',y'=: Zahlen, die durch die Wertigkeit und Häufigkeit der von Sauerstoff verschiedenen Elemente in II bestimmt werden und
- p,q =: Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}O_{x'}, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte Multimetalloxidmassen II sind solche, in denen Y¹ nur Wismut ist.

Unter diesen werden wiederum jene bevorzugt, die der allgemeinen Formel III,

[Bi_{a"}Z²_{b"}O_{x"}]_{p"} [Z²₁₂Z³_{c"}Z⁴_{d"}Fe_{e"}Z⁵_{f"}Z⁶_{g"}Z⁷_{h"}O_{y"}]_{q"} (III),

in der die Varianten folgende Bedeutung haben:
Z² = Molybdän oder Molybdän und Wolfram,
Z³ = Nickel und/oder Kobalt,
Z⁴ = Thallium, ein Alkalimetall und/oder ein Erdalkalimetall,
Z⁵ = Phosphor, Arsen, Bor, Antimon, Zinn, Cer und/oder Blei,
Z⁶ = Silicium, Aluminium, Titan und/oder Zirkonium,
Z⁷ = Kupfer, Silber und/oder Gold,
a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,
x",y"=Zahlen, die durch die Wertigkeit und Häufigkeit der von Sauerstoff verschiedenen Element in III bestimmt werden,
p",q"=Zahlen, deren Verhältnis p"/q" 0,1 bis 5, vorzugsweise 0,5 bis 2 beträgt,
entsprechen, wobei diejenigen Massen III ganz besonders bevorzugt werden, in denen Z²_{b"} = (Wolfram)_{b"} und Z²₁₂ = (Molybdän)₁₂ ist.

Ferner ist es von Vorteil, wenn wenigstens 25 mol-% (bevorzugt wenigstens 50 mol-% und besonders bevorzugt wenigstens 100 mol-%) des gesamten Anteils [Y¹_{a'}Y²_{b'}O_{x'}]ₚ ([Bi_{a"}Z²_{b"}O_{x"}]_{p"}) der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}O_{x'} [Bi_{a"}Z²_{b"}O_{x"}) vorliegen, deren Größtdurchmesser im Bereich 1 nm bis 100 µm liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z.B. in der EP-A 575897 sowie in der DE-A 19855913, der DE-A 10344149 und der DE-A 10344264 beschrieben.

Als Aktivmasse für die Katalysatoren des Katalysatorfestbetts 2 eignen sich bekanntermaßen Multimetalloxide, die die Elemente Mo und V enthalten.

Erfindungsgemäß geeignete, Mo und V enthaltende, Multimetalloxidaktivmassen können beispielsweise der US-A 3775474, der US-A 3954855, der US-A 3893951 und der US-A 4339355 oder der EP-A 614872 bzw. EP-A 1041062 oder der WO 03/055835 bzw. WO 03/057653 entnommen werden.

Insbesondere eignen sich auch die Multimetalloxidaktivmassen der DE-A 10325487 sowie der DE-A 10325488.

Ferner eignen sich in besonderer Weise die Multimetalloxidmassen der EP-A 427508, der DE-A 2909671, der DE-C 3151805, der DE-AS 2626887, der DE-A 4302991, der EP-A 700893, der EP-A 714700 und der DE-A 19736105 als Aktivmassen für für das erfindungsgemäße Verfahren geeignete Festbett-2-Katalysatoren. Besonders bevorzugt sind in diesem Zusammenhang die beispielhaften Ausführungsformen der EP-A 714 700 sowie der DE-A 19 73 6105.

Eine Vielzahl dieser für besagte Festbett-2-Katalysatoren geeigneten Multimetalloxidaktivmassen lässt sich unter der allgemeinen Formel IV,

Mo₁₂VₐX¹_{b}X²_{c}X³_{d}X⁴ₑX⁵_{f}X⁶_{g}Oₙ (IV),

in der die Variablen folgende Bedeutung haben:
- X¹ =: W, Nb, Ta, Cr und/oder Ce,
- X² =: Cu, Ni, Co, Fe, Mn und/oder Zn,
- X³ =: Sb und/oder Bi,
- X⁴ =: eines oder mehrere Alkalimetalle,
- X⁵ =: eines oder mehrere Erdalkalimetalle,
- X⁶ =: Si, Al, Ti und/oder Zr,
- a =: 1 bis 6,
- b =: 0,2 bis 4,
- c =: 0,5 bis 18,
- d =: 0 bis 40,
- e =: 0 bis 2,
- f =: 0 bis 4,
- g =: 0 bis 40 und
- n =: eine Zahl, die durch die Wertigkeit und Häufigkeit der von Sauerstoff verschiedenen Elemente in IV bestimmt wird,
subsummieren.

Bevorzugte Ausführungsformen innerhalb der aktiven Multimetalloxide IV sind jene, die von nachfolgenden Bedeutungen der Variablen der allgemeinen Formel IV erfasst werden:
- X¹ =: W, Nb, und/oder Cr,
- X² =: Cu, Ni, Co, und/oder Fe,
- X³ =: Sb,
- X⁴ =: Na und/oder K,
- X⁵ =: Ca, Sr und/oder Ba,
- X⁶ =: Si, Al, und/oder Ti,
- a =: 1,5 bis 5,
- b =: 0,5 bis 2,
- c =: 0,5 bis 3,
- d =: 0 bis 2,
- e =: 0 bis 0,2,
- f =: 0 bis 1 und
- n =: eine Zahl, die durch die Wertigkeit und Häufigkeit der von Sauerstoff verschiedenen Elemente in IV bestimmt wird.

Ganz besonders bevorzugte Multimetalloxide IV sind erfindungsgemäß jedoch jene der allgemeinen Formel V,

Mo₁₂V_{a'}Y¹_{b'}Y²_{c'}Y⁵_{f'}Y⁶_{g'}Oₙ (V),

mit
- Y¹ =: W und/oder Nb,
- Y² =: Cu und/oder Ni,
- Y⁵ =: Ca und/oder Sr,
- Y⁶ =: Si und/oder Al,
- a' =: 2 bis 4,
- b' =: 1 bis 1,5,
- c' =: 1 bis 3,
- f' =: 0 bis 0,5
- g' =: 0 bis 8 und
- n' =: eine Zahl, die durch die Wertigkeit und Häufigkeit der von Sauerstoff verschiedenen Elementen in V bestimmt wird.

Die erfindungsgemäß für Katalysatoren des Katalysatorfestbetts 2 geeigneten Multimetalloxidaktivmassen (IV) sind in sich bekannter, z.B. in der DE-A 4335973 oder in der EP-A 714700 offenbarter, Weise erhältlich. Insbesondere eignen sich aber auch die Multimetalloxidaktivmassen der DE-A 10 261 186.

Prinzipiell können für für das erfindungsgemäße Verfahren zu verwendende Festbett-2-Katalysatoren geeignete Multimetalloxidaktivmassen, insbesondere solche der allgemeinen Formel IV, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die für für das erfindungsgemäße Verfahren zu verwendende Festbett-2-Katalysatoren geeigneten Multimetalloxidaktivmassen, insbesondere jene der allgemeinen Formel IV, können für das erfindungsgemäße Verfahren sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 3 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für für das erfindungsgemäße Verfahren geeignete Festbett-2-Katalysatoren zu verwendende Multimetalloxidaktivmassen sind ferner Massen der allgemeinen Formel VI,

[D]ₚ[E]_{q} (VI),

in der die Variablen folgende Bedeutung haben:
- D =: Mo₁₂V_{a"}Z¹_{b"}Z²_{c"}Z³_{d"}Z⁴_{e"}Z⁵_{f"}Z⁶_{g"}O_{x"},
- E =: Z⁷₁₂Cu_{h"}H_{i"}O_{y"},
- Z¹ =: W, Nb, Ta, Cr und/oder Ce,
- Z² =: Cu, Ni, Co, Fe, Mn und/oder Zn,
- Z³ =: Sb und/oder Bi,
- Z⁴ =: Li, Na, K, Rb, Cs und/oder H
- Z⁵ =: Mg, Ca, Sr und/oder Ba,
- Z⁶ =: Si, Al, Ti und/oder Zr,
- Z⁷ =: Mo, W, V, Nb und/oder Ta,
- a" =: 1 bis 8,
- b" =: 0,2 bis 5,
- c" =: 0 bis 23,
- d" =: 0 bis 50,
- e" =: 0 bis 2,
- f" =: 0 bis 5,
- g" =: 0 bis 50,
- h" =: 4 bis 30,
- i" =: 0 bis 20 und
- x",y" =: Zahlen, die durch die Wertigkeit und Häufigkeit der von Sauerstoff verschiedenen Element in VI bestimmt werden und
- p,q =: von Null verschiedene Zahlen, deren Verhältnis p/q 160:1 bis 1:1 beträgt,
und die dadurch erhältlich sind, dass man eine Multimetalloxidmasse E,

Z⁷₁₂Cu_{h"}H_{i"}Q_{y"} (E),

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D,

Mo₁₂V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind jene Multimetalloxidaktivmassen VI, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen III-Katalysatoren enthalten z.B. die EP-A 668104, die DE-A 19736105 und die DE-A 19528646.

Hinsichtlich der Formgebung gilt bezüglich Multimetalloxidaktivmassen VI-Katalysatoren das bei den Multimetalloxidaktivmassen IV-Katalysatoren Gesagte.

Günstige Multimetalloxidaktivmassen für die Katalysatoren des Katalysatorfestbetts 2 des erfindungsgemäßen Verfahrens sind ferner Multielementoxidaktivmassen der allgemeinen Formel VII,

[A]ₚ[B]_{q}[C]ᵣ (VII),

in der die Variablen folgende Bedeutung haben:
- A =: Mo₁₂VₐX¹_{b}X²_{c}X³_{d}X⁴ₑX⁵_{f}X⁶_{g}Oₓ,
- B =: X₁⁷CuₕHᵢO_{y},
- C =: X₁⁸SbⱼHₖO_{z},
- X¹ =: W, Nb, Ta, Cr und/oder Ce, vorzugsweise W, Nb und/oder Cr,
- X² =: Cu, Ni, Co, Fe, Mn und/oder Zn, vorzugsweise Cu, Ni, Co und/oder Fe,
- X³ =: Sb und/oder Bi, vorzugsweise Sb,
- X⁴ =: Li, Na, K, Rb, Cs und/oder H. vorzugsweise Na und/oder K,
- X⁵ =: Mg, Ca, Sr und/oder Ba, vorzugsweise Ca, Sr und/oder Ba,
- X⁶ =: Si, Al, Ti und/oder Zr, vorzugsweise Si, Al und/oder Ti,
- X⁷ =: Mo, W, V, Nb und/oder Ta, vorzugsweise Mo und/oder W,
- X^{B} =: Cu, Ni, Zn, Co, Fe, Cd, Mn, Mg, Ca, Sr und/oder Ba, vorzugsweise Cu und/oder Zn, besonders bevorzugt Cu,
- a =: 1 bis 8, vorzugsweise 2 bis 6,
- b =: 0,2 bis 5, vorzugsweise 0,5 bis 2,5
- c =: 0 bis 23, vorzugsweise 0 bis 4,
- d =: 0 bis 50, vorzugsweise 0 bis 3,
- e =: 0 bis 2, vorzugsweise 0 bis 0,3,
- f =: 0 bis 5, vorzugsweise 0 bis 2,
- g =: 0 bis 50, vorzugsweise 0 bis 20,
- h =: 0,3 bis 2,5, vorzugsweise 0,5 bis 2, besonders bevorzugt 0,75 bis 1,5,
- i =: 0 bis 2, vorzugsweise 0 bis 1,
- j =: 0,1 bis 50, vorzugsweise 0,2 bis 20, besonders bevorzugt 0,2 bis 5,
- k =: 0 bis 50, vorzugsweise 0 bis 20, besonders bevorzugt 0 bis 12,
- x,y,z =: Zahlen, die durch die Wertigkeit und Häufigkeit der von Sauerstoff verschiedenen Elementen in A, B, C bestimmt werden,
- p, q =: positive Zahlen
- r =: 0 oder eine positive Zahl, vorzugsweise eine positive Zahl, wobei das Verhältnis p/(q+r) = 20:1 bis 1:20, vorzugsweise 5:1 bis 1:14 und besonders bevorzugt 2:1 bis 1:8 und , für den Fall, dass r eine positive Zahl ist, das Verhältnis q/r = 20:1 bis 1:20, vorzugsweise 4:1 bis 1:4, besonders bevorzugt 2:1 bis 1:2 und ganz besonders bevorzugt 1:1 beträgt,
die den Anteil [A]p in Form dreidimensional ausgedehnter Bereiche (Phasen) A der chemischen Zusammensetzung

A: Mo₁₂VaX¹_{b}X²_{c}X³_{d}X⁴ₑX⁵_{f}X⁶_{g}Oₓ,

den Anteil [B]q in Form dreidimensional ausgedehnter Bereiche (Phasen) B der chemischen Zusammensetzung

B: X₁⁷CuₕHⱼO_{y} und

den Anteil [C]ᵣ in Form dreidimensional ausgedehnter Bereiche (Phasen) C der chemischen Zusammensetzung

C: X₁⁸SbⱼHₖO_{z}

enthalten, wobei die Bereiche A, B und gegebenenfalls C relativ zueinander wie in einem Gemisch aus feinteiligem A, feinteiligem B und gegebenenfalls feinteiligem C verteilt sind, und wobei alle Variablen innerhalb der vorgegebenen Bereiche mit der Maßgabe auszuwählen sind, dass der molare Anteil des Elements Mo an der Gesamtmenge aller von Sauerstoff verschiedenen Elemente der Multielementoxidaktivmasse VII 20-mol% bis 80 mol-% beträgt, das molare Verhältnis von in der katalytisch aktiven Multielementoxidmasse VII enthaltenem Mo zu in der katalytisch aktiven Multielemetoxidmasse VII enthaltenem V, Mo/V, 15:1 bis 1:1 beträgt, das entsprechende molare Verhältnis Mo/Cu 30:1 bis 1:3 und das entsprechende molare Verhältnis Mo/(Gesamtmenge aus W und Nb) 80:1 bis 1:4 beträgt.

Bevorzugte Multielementoxidaktivmassen VII sind solche, deren Bereiche A eine Zusammensetzung im nachfolgenden Stöchiometrieraster der allgemeinen Formel VIII aufweisen,

Mo₁₂VₐX¹_{b}X²_{c}X⁵_{f}X⁶_{g}Oₓ (VIII),

mit
X¹ = W und/oder Nb,
X² = Cu und/oder Ni,
X⁵ = Ca und/oder Sr,
X⁶ = Si und/oder Al,
a = 2 bis 6,
b = 1 bis 2,
c = 1 bis 3,
f = 0 bis 0,75,
g = 0 bis 10, und
x = eine Zahl die durch die Wertigkeit und Häufigkeit der von Sauerstoff verschiedenen Elemente in (VIII) bestimmt wird.

Der im Zusammenhang mit den Multielementoxidaktivmassen VIII verwendete Begriff "Phase" meint dreidimensional ausgedehnte Bereiche, deren chemische Zusammensetzung von der ihrer Umgebung verschieden ist. Die Phasen sind nicht notwendigerweise röntgenographisch homogen. In der Regel bildet die Phase A eine kontinuierliche Phase, in der Partikel der Phase B und gegebenenfalls C dispergiert sind.

Die feinteiligen Phasen B und gegebenenfalls C bestehen mit Vorteil aus Partikeln, deren Größtdurchmesser, d.h., die längste durch den Schwerpunkt der Partikel gehende Verbindungsstrecke zweier auf der Oberfläche der Partikel befindlicher Punkte bis zu 300 µm, vorzugsweise 0,1 bis 200 µm, besonders bevorzugt 0,5 bis 50 µm und ganz besonders bevorzugt 1 bis 30 µm beträgt. Geeignet sind aber auch Partikel mit einem Größtdurchmesser von 10 bis 80 µm oder 75 bis 125 µm.

Prinzipiell können die Phasen A, B und gegebenenfalls C in den Multielementoxidaktivmassen VII amorph und/oder kristallin vorliegen.

Die den Multielementoxidaktivmassen der allgemeinen Formel VII zugrunde liegenden und anschließend zur Wandlung in Aktivmassen thermisch zu behandelnden innigen Trockengemische können z.B. so erhalten werden, wie es in den Schriften WO 02/24327, DE-A 4405514, DE-A 4440891, DE-A 19528646, DE-A 19740493, EP-A 756894, DE-A 19815280, DE-A 19815278, EP-A 774297, DE-A 19815281, EP-A 668104 und DE-A 19736105 beschrieben ist.

Das Grundprinzip der Herstellung von innigen Trockengemischen, die bei thermischer Behandlung zu Multielementoxidativmassen der allgemeinen Formel VII führen, besteht darin, wenigstens eine Multielementoxidmasse B (X₁⁷CuₕHᵢO_{y}) als Ausgangsmasse 1 und gegebenenfalls eine oder mehrere Multielementoxidmassen C (X₁⁸SbⱼHₖO_{z}) als Ausgangsmasse 2 entweder voneinander getrennt oder miteinander vergesellschaftet in feinteiliger Form vorzubilden und anschließend die Ausgangsmassen 1 und gegebenenfalls 2 mit einem Gemisch, das Quellen der elementaren Konstituenten der Multielementoxidmasse A

Mo₁₂VₐX_{b}¹X_{c}²X_{d}³Xₑ⁴X_{f}⁵X_{g}⁶Oₓ (A),

in einer der Stöchiometrie A entsprechenden Zusammensetzung enthält, im gewünschten Mengenverhältnis (gemäß der allgemeinen Formel VII) in innigen Kontakt zu bringen und die dabei resultierende innige Mischung gegebenenfalls zu trocknen.

Das innige in Kontakt bringen der Bestandteile der Ausgangsmassen 1 sowie gegebenenfalls 2 mit dem die Quellen der elementaren Konstituenten der Multimetalloxidmasse A enthaltenden Gemisch (Ausgangsmasse 3) kann sowohl trocken als auch nass erfolgen. Im letzteren Fall muss lediglich darauf geachtet werden, dass die vorgebildeten Phasen (Kristallite) B und gegebenenfalls C nicht in Lösung gehen. In wässrigem Medium ist letzteres bei pH-Werten, die nicht zu stark von 7 abweichen und bei nicht zu hohen Temperaturen üblicherweise gewährleistet. Erfolgt das innige in Kontakt bringen nass, wird abschließend normalerweise zum erfindungsgemäß thermisch zu behandelnden innigen Trockengemisch getrocknet (z.B. durch Sprühtrocknen). Im Rahmen eines trockenen Mischens fällt eine solche Trockenmasse automatisch an. Selbstredend können die feinteilig vorgebildeten Phasen B und gegebenenfalls C auch in ein plastisch verformbares Gemisch, das die Quellen der elementaren Konstituenten der Multimetalloxidmasse A enthält, eingearbeitet werden, wie es die DE-A 10046928 empfiehlt. Natürlich kann das innige in Kontakt bringen der Bestandteile der Ausgangsmassen 1 sowie gegebenenfalls 2 mit den Quellen der Multielementoxidmasse A (Ausgangsmasse 3) auch so erfolgen, wie es die DE-A 19815281 beschreibt.

Die thermische Behandlung zum Erhalt der Aktivmasse und die Formgebung kann wie bei den Multimetalloxidaktivmassen IV bis VI beschrieben erfolgen.

Ganz generell können Multimetalloxidaktivmassen IV bis VII-Katalysatoren mit Vorteil gemäß der Lehre der DE-A 10 325 487 bzw. DE-A 10 325 488 hergestellt werden.

Die Ausführung der ersten Reaktionsstufe des erfindungsgemäßen Verfahrens, vom Propen zum Acrolein, kann mit den für das Katalysatorfestbett 1 beschriebenen Katalysatoren in einfachster Weise und anwendungstechnisch zweckmäßig in einem mit den Festbett-1-Katalysatoren beschickten Rohrbündelreaktor, wie er z.B. in der EP-A 700 714 bzw. der DE-A 4 431 949 oder der WO 03/057653, oder der WO 03/055835, oder der WO 03/059857, oder der WO 03/076373 beschrieben ist, durchgeführt werden.

D.h., in einfachster Weise befindet sich das beim erfindungsgemäßen Verfahren zu verwendende Katalysatorfestbett 1 in den einheitlich beschickten Metallrohren eines Rohrbündelreaktors und um die Metallrohre wird ein Temperiermedium (Einzonenfahrweise), in der Regel eine Salzschmelze, geführt. Salzschmelze (Temperiermedium) und Reaktionsgasgemisch können dabei im einfachen Gleich- oder Gegenstrom geführt werden. Das Temperiermedium (die Salzschmelze) kann aber auch über den Reaktor betrachtet mäanderförmig um die Rohrbündel geführt werden, so dass lediglich über den gesamten Reaktor betrachtet ein Gleich- oder Gegenstrom zur Strömungsrichtung des Reaktionsgasgemischs besteht. Der Volumenstrom des Temperiermediums (Wärmeaustauschmittels) wird dabei üblicherweise so bemessen, dass der Temperaturanstieg (bedingt durch die Exothermie der Reaktion) des Wärmeaustauschmittels von der Eintrittstelle in den Reaktor bis zur Austrittstelle aus dem Reaktor 0 bis 10°C, häufig 2 bis 8°C, oft 3 bis 6°C beträgt. Die Eintrittstemperatur des Wärmeaustauschmittels in den Rohrbündelreaktor (sie entspricht in dieser Schrift der Temperatur des Katalysatorfestbetts 1) beträgt in der Regel 250 bis 450°C, häufig 300 bis 400°C bzw. 300 bis 380°C. Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle. Aber auch ionische Flüssigkeiten sind einsetzbar.

Zweckmäßigerweise wird das Reaktionsgasgemisch 1 der Beschickung mit Festbettkatalysator auf die gewünschte Reaktionstemperatur vorerwärmt zugeführt.

Insbesondere im Fall von angestrebten hohen (z.B. ≥ 140 Nl/l•h oder ≥ 160 Nl/l•h, in der Regel jedoch ≤ 600 Nl/l•h) Endbelastungen des Katalysatorfestbetts 1 mit Propen erfolgt die Durchführung des erfindungsgemäßen Verfahrens zweckmäßig in einem Zwei- oder Mehrzonenrohrbündelreaktor (eine Durchführung im Einzonenrohrbündelreaktor ist jedoch ebenfalls möglich). Eine bevorzugte Variante eines für diesen Zweck erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903582 offenbarten Zweizonenrohrbündelreaktoren sind geeignet. Eine Verfahrensbeschreibung gibt auch die EP-A 1106598.

D.h., in einfacher Weise befindet sich das erfindungsgemäß zu verwendende Katalysatorfestbett 1 in den einheitlich beschickten Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert eine Reaktionszone.

Vorzugsweise umströmt z.B. ein Salzbad A denjenigen Abschnitt der Rohre (die Reaktionszone A), in welchem sich die oxidative Umsetzung des Propens (beim einfachen Durchgang) bis zum Erreichen eines Umsatzwertes im Bereich von 40 bis 80 mol.% vollzieht und ein Salzbad B umströmt den Abschnitt der Rohre (die Reaktionszone B), in welchem sich die oxidative Anschlußumsetzung des Propens (beim einfachen Durchgang) bis zum Erreichen eines Umsatzwertes von in der Regel wenigstens 93 mol.% vollzieht (bei Bedarf können sich an die Reaktionszonen A, B weitere Reaktionszonen anschließen, die auf individuellen Temperaturen gehalten werden).

Innerhalb der jeweiligen Temperaturzone kann das Salzbad prinzipiell wie bei der Einzonenfahrweise geführt werden. Die Eintrittstemperatur des Salzbades B liegt normalerweise wenigstens 5 bis 10°C oberhalb der Temperatur des Salzbades A. Im übrigen können die Eintrittstemperaturen im für die Einzonenfahrweise empfohlenen Temperaturbereich für die Eintrittstemperatur liegen.

Ansonsten kann die Zwei-Zonen-Hochlastfahrweise z.B. wie in der DE-A 10308836, der EP-A 1106598, oder wie in der WO 01/36364, oder der DE-A 19927624, oder der DE-A 19948523, der DE-A 10313210, der DE-A 10313213 oder wie in der DE-A 19948248 beschrieben durchgeführt werden.

Danach eignet sich das erfindungsgemäße Verfahren für Propenbelastungen des Katalysatorfestbetts 1 von ≥ 70 Nl/l•h, ≥ 90 Nl/l•h, ≥ 110 Nl/l•h, ≥ 130 Nl/l•h, ≥ 140 Nl/l•h, ≥ 160 Nl/l•h, ≥ 180 Nl/l•h, ≥ 240 Nl/l•h, ≥ 300 Nl/l•h, jedoch normalerweise ≤ 600 Nl/l•h. Hier (d.h., generell bei Propenbelastungen in dieser Schrift), anders als sonst in dieser Schrift, ist die Belastung auf das Volumen des Katalysatorfestbetts 1 ausschließlich gegebenenfalls mitverwendeter Abschnitte die ausschließlich aus Inertmaterial bestehen bezogen.

Zur Bereitung des Katalysatorfestbetts 1 können beim erfindungsgemäßen Verfahren nur die entsprechende Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation von Propen zu Acrolein im wesentlichen inert verhaltenden (aus Inertmaterial bestehenden), Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete Erststufen-Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Erststufen-Katalysatorformkörper entspricht.

In der Regel ist es günstig, wenn sich die chemische Zusammensetzung der verwendeten Aktivmasse über das Katalysatorfestbett 1 nicht verändert. D.h., die für einen einzelnen Katalysatorformkörper verwendete Aktivmasse kann zwar ein Gemisch aus verschiedenen, die Elemente Mo und/oder W sowie wenigstens eines der Elemente Bi, Fe, Sb, Sn und Cu enthaltenden, Multimetalloxiden sein, für alle Katalysatorformkörper des Katalysatorfestbetts 1 ist dann jedoch vorteilhaft das gleiche Gemisch zu verwenden.

Bevorzugt nimmt normalerweise die volumenspezifische (d.h., die auf die Einheit des Volumens normierte) Aktivität innerhalb des Katalysatorfestbetts 1 in Strömungsrichtung des Reaktionsgasausgangsgemischs kontinuierlich, abrupt oder stufenförmig zu.

Die volumenspezifische Aktivität kann dabei z.B. in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen des Festbetts enthaltene Aktivmasse bzw. Katalysatoraktivität.

Eine in Strömungsrichtung des Reaktionsgasgemischs über des Katalysatorfestbett 1 wenigstens einmal zunehmende volumenspezifische Aktivität lässt sich für das erfindungsgemäße Verfahren somit in einfacher Weise z.B. dadurch einstellen, dass man die Schüttung mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Eine Zunahme der volumenspezifischen Aktivität ist aber auch z.B. dadurch möglich, dass man bei gleich bleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Natürlich können für das Katalysatorfestbett 1 aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum mit inerten Verdünnungskörpern verdünnt werden.

Vorab und/oder im Anschluss an die Aktivmasse aufweisenden Abschnitte des Katalysatorfestbetts 1 können sich ausschließlich aus Inertmaterial (z.B. nur Verdünnungsformkörpern) bestehende Schüttungen befinden (sie werden in dieser Schrift begrifflich, soweit nichts anderes gesagt wird, dem Katalysatorfestbett zugerechnet). Diese können ebenfalls auf die Temperatur des Katalysatorfestbetts 2 gebracht sein. Dabei können die für die Inertschüttung verwendeten Verdünnungsformkörper die gleiche Geometrie wie die zu den Aktivmasse aufweisenden Abschnitte des Katalysatorfestbetts 1 verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4-5 mm auf.

Vielfach ist beim erfindungsgemäßen Verfahren der Aktivmasse aufweisende Abschnitt des Katalysatorfestbetts 1 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge des Aktivmasse aufweisenden Abschnitts der Festbett-1-Katalysatorschüttung, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, vorzugsweise 10 bis 40 Gew.-% oder 20 bis 40 Gew.-% und besonders bevorzugt 25 bis 35 Gew.-% beträgt. Im Anschluss an diese erste Zone befindet sich dann häufig vorteilhaft bis zum Ende der Länge des Aktivmasse aufweisenden Abschnitts des Katalysatorfestbetts 1 (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige Schüttung derselben Katalysatorformkörper, die auch in der ersten Zone verwendet worden sind.

Das Vorgenannte trifft insbesondere dann zu, wenn im Katalysatorfestbett 1 als Katalysatorformkörper Vollkatalysatorringe oder Schalenkatalysatorringe eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-%-Punkte tiefer liegt, als der Aktivmassenanteil der Schalenkatalysatorformkörper am Ende des Katalysatorfestbetts.

Eine reine Inertmaterialschüttung, deren Länge, bezogen auf die Gesamtlänge des Katalysatorfestbetts 1, zweckmäßig 1 oder 5 bis 20 % beträgt, leitet in Strömungsrichtung des Reaktionsgasgemischs in der Regel das Katalysatorfestbett 1 ein. Sie wird normalerweise als Aufheizzone für das Reaktionsgasgemisch genutzt.

Üblicherweise sind die Kontaktrohre in den Rohrbündelreaktoren für die erste Stufe aus ferritischem Stahl gefertigt und weisen in typischer Weise eine Wanddicke von 1 bis 3 mm auf. Ihr Innendurchmesser beträgt in der Regel (einheitlich) 20 bis 30 mm, häufig 21 bis 26 mm. Anwendungstechnisch zweckmäßig beläuft sich die im Rohrbündelbehälter untergebrachte Anzahl an Kontaktrohen auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden für die erste Reaktionsstufe eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Die Ausführung der zweiten Reaktionsstufe des erfindungsgemäßen Verfahrens, vom Acrolein zur Acrylsäure, kann mit den für das Katalysatorfestbett 2 beschriebenen Katalysatoren in einfachster Weise und anwendungstechnisch zweckmäßig in einem mit den Festbett-2-Katalysatoren beschickten Rohrbündelreaktor, wie er z.B. in der EP-A 700 893 bzw. der DE-A 4 431 949 oder der WO 03/057653, oder der WO 03/055835, oder der WO 03/059857, oder der WO 03/076373 beschrieben ist, durchgeführt werden.

D.h., in einfachster Weise befindet sich das beim erfindungsgemäßen Verfahren zu verwendende Katalysatorfestbett 2 in den einheitlich beschickten Metallrohren eines Rohrbündelreaktors und um die Metallrohre wird ein Temperiermedium (Einzonenfahrweise), in der Regel eine Salzschmelze, geführt. Salzschmelze (Temperiermedium) und Reaktionsgasgemisch können dabei im einfachen Gleich- oder Gegenstrom geführt werden. Das Temperiermedium (die Salzschmelze) kann aber auch über den Reaktor betrachtet mäanderförmig um die Rohrbündel geführt werden, so dass lediglich über den gesamten Reaktor betrachtet ein Gleich- oder Gegenstrom zur Strömungsrichtung des Reaktionsgasgemischs besteht. Der Volumenstrom des Temperiermediums (Wärmeaustauschmittels) wird dabei üblicherweise so bemessen, dass der Temperaturanstieg (bedingt durch die Exothermie der Reaktion) des Wärmeaustauschmittels von der Eintrittstelle in den Reaktor bis zur Austrittstelle aus dem Reaktor 0 bis 10°C, häufig 2 bis 8°C, oft 3 bis 6°C beträgt. Die Eintrittstemperatur des Wärmeaustauschmittels in den Rohrbündelreaktor (sie entspricht in dieser Schrift der Temperatur des Katalysatorfestbetts) beträgt in der Regel 220 bis 350°C, häufig 245 bis 285°C bzw. 245 bis 265°C. Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle. Aber auch ionische Flüssigkeiten sind einsetzbar.

Zweckmäßigerweise wird das Reaktionsgasgemisch 2 der Beschickung mit Festbettkatalysator auf die gewünschte Reaktionstemperatur vorerwärmt zugeführt.

Insbesondere im Fall von angestrebten hohen (z.B. ≥ 140 Nl/l•h, in der Regel jedoch ≤ 600 Nl/l•h) Endbelastungen des Katalysatorfestbetts 2 mit Acrolein erfolgt die Durchführung des erfindungsgemäßen Verfahrens zweckmäßig in einem Zwei- oder Mehrzonenrohrbündelreaktor (eine Durchführung im Einzonenrohrbündelreaktor ist jedoch ebenfalls möglich). Eine bevorzugte Variante eines für diesen Zweck erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903582 offenbarten Zweizonenrohrbündelreaktoren sind geeignet.

D.h., in einfacher Weise befindet sich das erfindungsgemäß zu verwendende Katalysatorfestbett 2 in den einheitlich beschickten Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert eine Temperatur- bzw. Reaktionszone.

Vorzugsweise umströmt z.B. ein Salzbad C denjenigen Abschnitt der Rohre (die Reaktionszone C), in welchem sich die oxidative Umsetzung des Acroleins (beim einfachen Durchgang) bis zum Erreichen eines Umsatzwertes im Bereich von 55 bis 85 mol.% vollzieht und ein Salzbad D umströmt den Abschnitt der Rohre (die Rekationszone D), in welchem sich die oxidative Anschlußumsetzung des Acroleins (beim einfachen Durchgang) bis zum Erreichen eines Umsatzwertes von in der Regel wenigstens 90 mol.% vollzieht (bei Bedarf können sich an die Reaktionszonen C, D weitere Reaktionszonen anschließen, die auf individuellen Temperaturen gehalten werden).

Innerhalb der jeweiligen Temperaturzone kann das Salzbad prinzipiell wie bei der Einzonenfahrweise geführt werden. Die Eintrittstemperatur des Salzbades D liegt normalerweise wenigstens 5 bis 10°C oberhalb der Temperatur des Salzbades C. Im übrigen können die Eintrittstemperaturen im für die Einzonenfahrweise empfohlenen Temperaturbereich für die Eintrittstemperatur liegen.

Ansonsten kann die Zwei-Zonen-Hochlastfahrweise z.B. wie in der DE-A 19948523, der EP-A 1106598 oder wie in der DE-A 19948248 beschrieben durchgeführt werden.

Danach eignet sich das erfindungsgemäße Verfahren für Acroleinbelastungen des Katalysatorfestbetts 2 von ≥ 70 Nl/l•h, ≥ 90 Nl/l•h, ≥ 110 Nl/l•h, ≥ 130 Nl/l•h, ≥ 180 Nl/l•h, ≥ 240 Nl/l•h, ≥ 300 Nl/l•h, jedoch normalerweise ≤ 600 Nl/l•h. Hier (d.h., generell bei Acroleinbelastungen in dieser Schrift), anders als sonst in dieser Schrift, ist die Belastung auf das Volumen des Katalysatorfestbetts 2 ausschließlich gegebenenfalls mitverwendeter Abschnitte die ausschließlich aus Inertmaterial bestehen bezogen.

Zur Bereitung des Katalysatorfestbetts 2 können beim erfindungsgemäßen Verfahren nur die entsprechende Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation im wesentlichen inert verhaltenden (aus Inertmaterial bestehenden), Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete Zweitstufen-Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

In der Regel ist es günstig, wenn sich die chemische Zusammensetzung der verwendeten Aktivmasse über das Katalysatorfestbett 2 nicht verändert. D.h., die für einen einzelnen Katalysatorformkörper verwendete Aktivmasse kann zwar ein Gemisch aus verschiedenen die Elemente Mo und V enthaltenden Multimetalloxiden sein, für alle Katalysatorformkörper des Katalysatorfestbetts 2 ist dann jedoch vorteilhaft das gleiche Gemisch zu verwenden.

Bevorzugt nimmt normalerweise die volumenspezifische (d.h., die auf die Einheit des Volumens normierte) Aktivität innerhalb des Katalysatorfestbetts 2 in Strömungsrichtung des Reaktionsgasgemischs kontinuierlich, abrupt oder stufenförmig zu.

Die volumenspezifische Aktivität kann dabei z.B. in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper gewählt wird, desto geringer ist die in einem bestimmten Volumen des Festbetts enthaltene Aktivmasse bzw. Katalysatoraktivität.

Eine in Strömungsrichtung des Reaktionsgasgemischs über des Katalysatorfestbett 2 wenigstens einmal zunehmende volumenspezifische Aktivität lässt sich für das erfindungsgemäße Verfahren somit in einfacher Weise z.B. dadurch einstellen, dass man die Schüttung 2 mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Eine Zunahme der volumenspezifischen Aktivität ist aber auch z.B. dadurch möglich, dass man bei gleich bleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Natürlich können für das Katalysatorfestbett 2 aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum mit inerten Verdünnungskörpern verdünnt werden.

Vorab und/oder im Anschluss an die Aktivmasse aufweisenden Abschnitte des Katalysatorfestbetts 2 können sich ausschließlich aus Inertmaterial (z.B. nur Verdünnungsformkörpern) bestehende Schüttungen befinden (sie werden in dieser Schrift begrifflich, soweit nichts anderes gesagt wird, dem Katalysatorfestbett 2 zugerechnet). Diese können ebenfalls auf die Temperatur des Katalysatorfestbetts 2 gebracht sein. Dabei können die für die Inertschüttung verwendeten Verdünnungsformkörper die gleiche Geometrie wie die zu den Aktivmasse aufweisenden Abschnitte des Katalysatorfestbetts 2 verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4-5 mm auf.

Vielfach ist beim erfindungsgemäßen Verfahren der Aktivmasse aufweisende Abschnitt des Katalysatorfestbetts 2 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge des Aktivmasse aufweisenden Abschnitts der Festbett-2-Katalysatorschüttung, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 10 bis 50 Gew.-%, vorzugsweise 20 bis 45 Gew.-% und besonders bevorzugt 25 bis 35 Gew.-% beträgt. Im Anschluss an diese erste Zone befindet sich dann häufig vorteilhaft bis zum Ende der Länge des Aktivmasse aufweisenden Abschnitts des Katalysatorfestbetts 2 (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige Schüttung derselben Katalysatorformkörper, die auch in der ersten Zone verwendet worden sind.

Das Vorgenannte trifft insbesondere dann zu, wenn im Katalysatorfestbett 2 als Katalysatorformkörper Schalenkatalysatorringe oder Schalenkatalysatorkugeln eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-%-Punkte tiefer liegt, als der Aktivmassenanteil der Schalenkatalysatorformkörper am Ende des Katalysatorfestbetts 2.

Eine reine Inertmaterialschüttung, deren Länge, bezogen auf die Gesamtlänge des Katalysatorfestbetts, zweckmäßig 5 bis 20 % beträgt, leitet in Strömungsrichtung des Reaktionsgasgemischs in der Regel das Katalysatorfestbett 2 ein. Sie wird normalerweise als Aufheizzone für das Reaktionsgasgemisch genutzt.

Üblicherweise sind die Kontaktrohre in den Zweitstufen-Rohrbündelreaktoren aus ferritischem Stahl gefertigt und weisen in typischer Weise eine Wanddicke von 1 bis 3 mm auf. Ihr Innendurchmesser beträgt in der Regel (einheitlich) 20 bis 30 mm, häufig 21 bis 26 mm. Anwendungstechnisch zweckmäßig beläuft sich die im Rohrbündelbehälter untergebrachte Anzahl an Kontaktrohen auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden für die erste Reaktionsstufe eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Wie bereits beschrieben, können beim erfindungsgemäßen Verfahren beide Reaktionsstufen in Einzonen- oder in Zweizonenrohrbündelreaktoren durchgeführt werden. Selbstredend kann aber auch nur die erste Reaktionsstufe in einem Einzonenrohrbündelreaktor und die zweite Reaktionsstufe in einem Zweizonenrohrbündelreaktor (oder umgekehrt) durchgeführt werden.

Zwischen den Rohrbündelreaktoren der ersten und der zweiten Reaktionsstufe kann sich ein Zwischenkühler befinden, der gegebenenfalls Inertschüttungen enthalten kann.

Selbstredend können für das erfindungsgemäße Verfahren das Katalysatorfestbett 1 und das Katalysatorfestbett 2 auch räumlich aufeinanderfolgend in einem einzigen, ebenfalls z.B. zwei Temperaturzonen aufweisenden, Vielkontaktrohrrohrbündelreaktor untergebracht sein, wie es z.B. die WO 03/059857, die EP-A 911313 und die EP-A 990636 beschreiben. Man spricht in diesem Fall von einem Single-Reactor-Zweistufenverfahren. Eine Temperaturzone erstreckt sich dabei in der Regel über ein Katalysatorfestbett. Zwischen den beiden Katalysatorfestbetten kann sich zusätzlich eine Inertschüttung befinden, die gegebenenfalls in einer dritten Temperaturzone befindlich ist und separat temperiert wird.

Das für das Beschickungsgasgemisch der ersten Reaktionsstufe (das Reaktionsgasausgangsgemisch 1) beim erfindungsgemäßen Verfahren zu verwendende Inertgas kann unabhängig von der für das Katalysatorfestbett 1 gewählten Propenbelastung zu z.B. ≥ 20 Vol.-%, oder zu ≥ 30 Vol.-%, oder zu ≥ 40 Vol.-%, oder zu ≥ 50 Vol.-%, oder zu ≥ 60 Vol.-%, oder zu ≥ 70 Vol.-%, oder zu ≥ 80 Vol.-%, oder zu ≥ 90 Vol.-%, oder zu ≥ 95 Vol.-% aus molekularem Stickstoff bestehen.

Das inerte Verdünnungsgas kann aber auch z.B. zu 2 bis 35 bzw. 20 Gew.-% aus H₂O und zu 65 bis 98 Vol.-% aus N₂ bestehen.

Bei Propenbelastungen des Katalysatorfestbetts 1 oberhalb von 250 Nl/l•h wird für das erfindungsgemäße Verfahren jedoch die Mitverwendung von inerten Verdünnungsgasen wie Propan, Ethan, Methan, Butan, Pentan, CO₂, CO, Wasserdampf und/oder Edelgasen empfohlen. Selbstverständlich können diese Gase aber auch bereits bei geringeren Propenbelastungen mitverwendet werden.

Der Arbeitsdruck kann bei der erfindungsgemäßen Gasphasenpartialoxidation des Propens in der ersten Reaktionsstufe sowohl unterhalb von Normaldruck (z.B. bis zu 0,5 bar) als auch oberhalb von Normaldruck liegen. Typischerweise wird der Arbeitsdruck bei der Gasphasen-Partialoxidation des Propens bei Werten von 1 bis 5 bar, häufig 1 bis 3 bar liegen.

Normalerweise wird der Reaktionsdruck bei der erfindungsgemäßen Propenpartialoxidation zu Acrolein 100 bar nicht überschreiten.

Das molare Verhältnis von O₂:Propen im Reaktionsgasausgangsgemisch 1, das beim erfindungsgemäßen Verfahren durch das Katalysatorfestbett 1 geführt wird, wird normalerweise ≥ 1 betragen. Üblicherweise wird dieses Verhältnis bei Werten ≤ 3 liegen. Häufig wird das molare Verhältnis von O₂:Propen im vorgenannten Beschickungsgasgemisch erfindungsgemäß 1 bis 2 bzw. 1 bis 1,5 betragen. Vielfach wird man das erfindungsgemäße Verfahren mit einem im Reaktionsgasausgangsgemisch 1 vorliegenden Propen:Sauerstoff:Inertgas (einschließlich Wasserdampf) - Volumenverhältnis (NI). von 1 :(1 bis 3) : (3 bis 30), vorzugsweise von 1 :(1,5 bis 2,3) : (10 bis 15) ausführen.

Der Propenanteil im Reaktionsgasausgangsgemisch 1 kann z.B. bei Werten von 4 bis 20 Vol.-%, häufig bei 5 oder 7 bis 15 Vol.-% bzw. 6 oder 8 bis 12 Vol.-% oder 5 bis 8 Vol.-% liegen (jeweils bezogen auf das Gesamtvolumen).

Eine typische Zusammensetzung des Reaktionsgasausgangsgemischs 1 (unabhängig von der gewählten Belastung) kann für die erste Reaktionsstufe z.B. die nachfolgenden Komponenten enthalten:
6 bis 6,5 Vol.-% Propen,
3 bis 3,5 Vol.-% H₂O,
0,3 bis 0,5 Vol.-% CO,
0,8 bis 1,2 Vol.-% CO₂,
0,025 bis 0,04 Vol.-% Acrolein,
10,4 bis 10,7 Vol.-%O₂ und
als Restmenge ad 100% molekularer Stickstoff,

oder: 5,4 Vol.-% Propen,
10,5 Vol.-% Sauerstoff,
1,2 Vol.-% COₓ,
80,5 Vol.-% N₂ und
2,4 Vol.-% H₂O.

Das Reaktionsgasausgangsgemisch 1 kann für die erste Reaktionsstufe aber auch wie folgt zusammengesetzt sein:
6 bis 15 Vol.-% Propen,
4 bis 30 Vol.-% (häufig 6 bis 15 Vol.-%) Wasser,
≥ 0 bis 10 Vol.-% (vorzugsweise ≥ 0 bis 5 Vol.-%) an von Propen, Wasser, Sauerstoff und Stickstoff verschiedenen Bestandteilen, soviel molekularem Sauerstoff, dass das molare Verhältnis von enthaltenem molekularem Sauerstoff zu enthaltenem molekularem Propen 1,5 bis 2,5 beträgt und als Restmenge bis zur 100 Vol.-% Gesamtmenge aus molekularem Stickstoff.

Eine andere mögliche Reaktionsgasausgangsgemisch-1-Zusammensetzung kann für die erste Reaktionsstufe enthalten:
6,0 Vol.-% Propen,
60 Vol.-% Luft und
34 Vol.-% H₂O.

Alternativ können auch Reaktionsgasausgangsgemische 1 der Zusammensetzung gemäß Example 1 der EP-A 990 636, oder gemäß Example 2 der EP-A 990 636, oder gemäß Example 3 der EP-A 1 106 598, oder gemäß Example 26 der EP-A 1 106 598, oder gemäß Example 53 der EP-A 1 106 598 für die erste Reaktionsstufe verwendet werden.

Weitere erfindungsgemäß geeignete Reaktionsgasausgangsgemische 1 für die erste Reaktionsstufe können im nachfolgenden Zusammensetzungsraster liegen:
7 bis 11 Vol.-% Propen,
6 bis 12 Vol.-% Wasser,
≥ 0 bis 5 Vol.-% an von Propen, Wasser, Sauerstoff und Stickstoff verschiedenen Bestandteilen,
soviel molekularem Sauerstoff, dass das molare Verhältnis von enthaltenem Sauerstoff zu enthaltenem molekularem Propen 1,4 bis 2,2 beträgt, und
als Restmenge bis zur 100 Vol.-% Gesamtmenge molekularer Stickstoff.

Als im Reaktionsgasausgangsgemisch 1 zu verwendendes Propen kommen vor allem polymer grade Propen und chemical grade Propen in Betracht, wie es z.B. die DE-A 10232748 beschreibt.

An dieser Stelle sei noch erwähnt, dass ein Teil des Beschickungsgasgemischs der ersten Stufe ("Propen → Acrolein") sogenanntes Kreisgas sein kann. Hierbei handelt es sich um Gas, das nach der Produktabtrennung in der der zweiten Reaktionsstufe üblicherweise folgenden Aufarbeitung (Acrylsäureabtrennung) vom Produktgasgemisch der zweiten Reaktionsstufe verbleibt und in der Regel zum Teil als weitgehend inertes Verdünnungsgas zum Beschicken der ersten und/oder zweiten Reaktionsstufe rückgeführt wird.

Als Sauerstoffquelle wird normalerweise Luft eingesetzt.

Die Belastung des Katalysatorfestbetts 1 (hier ausschließlich reiner Inertabschnitte) mit Reaktionsgasausgangsgemisch 1 wird beim erfindungsgemäßen Verfahren in typischer Weise 1000 bis 10000 Nl/l•h, meist 1000 bis 5000 Nl/l•h, häufig 1500 bis 4000 Nl/l•h betragen.

Gegebenenfalls nach erfolgter Zwischenkühlung wird das Produktgasgemisch der ersten Reaktionsstufe der zweiten Reaktionsstufe zugeführt. Der in der zweiten Reaktionsstufe benötigte Sauerstoff kann dabei bereits als Überschuß dem Reaktionsgasausgangsgemisch 1 für die erste Reaktionsstufe zugesetzt werden und somit Bestandteil des Produktgasgemischs der ersten Reaktionsstufe sein. In diesem Fall kann das, gegebenenfalls zwischengekühlte, Produktgasgemisch der ersten Reaktionsstufe unmittelbar das Beschickungsgasgemisch der zweiten Reaktionsstufe sein. Der für den zweiten Oxidationsschritt vom Acrolein zur Acrylsäure benötigte Sauerstoff kann aber dem Produktgasgemisch der ersten Reaktionsstufe auch vor dem Eintritt desselben in die zweite Reaktionsstufe erst teilweise oder vollständig zugesetzt werden, z.B. in Form vom Luft. Mit dieser Zugabe kann dabei eine Direktkühlung des Produktgasgemischs der ersten Reaktionsstufe verbunden sein.

Aus dem vorgenannten Zusammenhang resultierend, kann das im Beschickungsgasgemisch für die zweite Reaktionsstufe (dem Reaktionsgasausgangsgemisch 2) enthaltene Inertgas zu z.B. ≥ 20 Vol.-%, oder zu ≥ 30 Vol.-%, oder zu ≥ 40 Vol.-%, oder zu ≥ 50 Vol.%, oder zu ≥ 60 Vol.-%, oder zu ≥ 70 Vol.-%, oder zu ≥ 80 Vol.-%, oder zu ≥ 90 Vol.-%, oder zu ≥ 95 Vol.-% aus molekularem Stickstoff bestehen.

Häufig wird das inerte Verdünnungsgas im Beschickungsgas für die zweite Reaktionsstufe aber zu 5 bis 25 bzw. 20 Gew.-% aus H₂O (wird in der ersten Reaktionsstufe gebildet und gegebenenfalls zugesetzt) und zu 70 bis 90 Vol.-% aus N₂ bestehen.

Bei Acroleinbelastungen des Katalysatorfestbetts 2 oberhalb von 250 Nl/l•h wird für das erfindungsgemäße Verfahren jedoch die Mitverwendung von inerten Verdünnungsgasen wie Propan, Ethan, Methan, Butan, Pentan, CO₂, Wasserdampf und/oder Edelgasen empfohlen. Selbstverständlich können diese Gase aber auch bereits bei geringeren Acroleinbelastungen mitverwendet werden.

Der Arbeitsdruck kann bei der erfindungsgemäßen Gasphasenpartialoxidation des Acroleins sowohl unterhalb von Normaldruck (z.B. bis zu 0,5 bar) als auch oberhalb von Normaldruck liegen. Typischerweise wird der Arbeitsdruck bei der GasphasenPartialoxidation des Acroleins bei Werten von 1 bis 5 bar, häufig 1 bis 3 bar liegen.

Normalerweise wird der Reaktionsdruck bei der erfindungsgemäßen Acroleinpartialoxidation 100 bar nicht überschreiten.

Das molare Verhältnis von O₂:Acrolein im Beschickungsgasgemisch für die zweite Reaktionsstufe, das beim erfindungsgemäßen Verfahren durch das Katalysatorfestbett 2 geführt wird, wird normalerweise ≥ 1 betragen. Üblicherweise wird dieses Verhältnis bei Werten ≤ 3 liegen. Häufig wird das molare Verhältnis von O₂:Acrolein im vorgenannten Beschickungsgasgemisch erfindungsgemäß 1 bis 2 bzw. 1 bis 1,5 betragen. Vielfach wird man das erfindungsgemäße Verfahren mit einem im Reaktionsgasausgangsgemisch 2 (Beschickungsgasgemisch für die zweite Reaktionsstufe) vorliegenden Acrolein:Sauerstoff:Wasserdampf:lnertgas - Volumenverhältnis (Nl) von 1 :(1 bis 3). : (0 bis 20) : (3 bis 30), vorzugsweise von 1:(1 bis 3) : (0,5 bis 10) : (7 bis 20) ausführen.

Der Acroleinanteil im Beschickungsgasgemisch für die zweite Reaktionsstufe kann z.B. bei Werten von 3 oder 6 bis 15 Vol.-%, häufig bei 4 oder 6 bis 10 Vol.-% bzw. 5 bis 8 Vol.-% liegen (jeweils bezogen auf das Gesamtvolumen). Die Belastung des Katalysatorfestbetts 2 (hier ausschließlich reiner Inertabschnitte) mit Beschickungsgasgemisch (Reaktionsgasausgangsgemisch 2) wird beim erfindungsgemäßen Verfahren in typischer Weise wie für die erste Reaktionsstufe 1000 bis 10000 Nl/l•h, meist 1000 bis 5000 Nl/l•h, häufig 1500 bis 4000 Nl/l•h betragen.

Bei der Ausübung des erfindungsgemäßen Verfahrens wird man ein frisches Katalysatorfestbett 1 nach seiner Formierung normalerweise so betreiben, dass man nach Festlegung der Zusammensetzung des Reaktionsgasausgangsgemischs 1 und Festlegung der Belastung des Katalysatorfestbetts 1 mit Reaktionsgasausgangsgemisch 1 die Temperatur des Katalysatorfestbetts 1 (bzw. die Eintrittstemperatur des Temperiermediums in die Temperierzone des Rohrbündelreaktors) so einstellt, dass der Umsatz U^{Pro} des Propens bei einmaligem Durchgang des Reaktionsgasgemischs 1 durch das Katalysatorfestbett 1 wenigstens 93 mol.-% beträgt. Bei Verwendung günstiger Katalysatoren sind auch Werte für U^{Pro} ≥ 94 mol.-%, oder ≥ 95 mol.-%, oder ≥ 96 mol.-%, oder ≥ 97 mol.-% und häufig sogar mehr möglich.

Bei kontinuierlicher Ausübung der heterogen katalysierten Partialoxidation wird man die Zusammensetzung des Reaktionsgasausgangsgemischs 1 und die Belastung des Katalysatorfestbetts 1 mit Reaktionsgasausgangsgemisch 1 im wesentlichen konstant beibehalten (gegebenenfalls wird die Belastung an die fluktuierende Marktnachfrage angepasst). Einem Abfallen der Aktivität des Katalysatorfestbetts 1 über die Zeit wird man unter diesen Produktionsbedingungen normalerweise dadurch entgegenwirken, dass man von Zeit zu Zeit die Temperatur des Katalysatorfestbetts (die Eintrittstemperatur des Temperiermediums in die Temperaturzone des Rohrbündelreaktors) erhöht (die Fließgeschwindigkeit des Temperiermediums wird normalerweise im wesentlichen ebenfalls beibehalten), um den Propenumsatz bei einmaligem Durchgang des Reaktionsgasgemischs im angestrebten Zielkorridor (d.h., bei U^{Pro} ≥ 93 mol.-%, bzw. ≥ 94 mole.-%, bzw. ≥ 95 mol.-%, bzw. ≥ 96 mol.-%, bzw. ≥ 97 mol.-%) zu halten. Eine solche Vorgehensweise zieht jedoch die Eingangs dieser Schrift beschriebenen Nachteile mit sich.

Man wird deshalb so verfahren, dass man die Gasphasenpartialoxidation erfindungsgemäß wenigstens einmal innerhalb von 7500 Betriebsstunden unterbricht, um bei einer Temperatur des Katalysatorfestbetts 1 von 250 bis 550°C ein aus molekularem Sauerstoff, Inertgas und gegebenenfalls Wasserdampf bestehendes Gasgemisch G durch das Katalysatorfestbett 1 zu führen. Anschließend wird die Partialoxidation unter weitgehendem Beibehalt der Verfahrensbedingungen fortgesetzt (die Wiederherstellung der Propenbelastung des Katalysatorfestbetts 1 erfolgt vorzugsweise langsam) und die Temperatur des Katalysatorfestbetts 1 so eingestellt, dass der Propenumsatz den angestrebten Zielwert erreicht. In der Regel wird dieser Temperaturwert bei gleichem Umsatz bei einem etwas niedrigeren Wert liegen als die Temperatur, die das Katalysatorfestbett 1 vor der Unterbrechung der Partialoxidation und der erfindungsgemäßen Behandlung mit dem Gasgemisch G aufwies. Von diesem Temperaturwert des Katalysatorfestbetts 1 ausgehend wird die Partialoxidation unter weitgehendem Beibehalt der übrigen Bedingungen fortgesetzt und dabei dem Abfall der Aktivität des Katalysatorfestbetts 1 über die Zeit in zweckmäßiger Weise wiederum dadurch entgegengewirkt, dass man von Zeit zu Zeit die Temperatur des Katalysatorfestbetts 1 erhöht. Innerhalb z.B. eines darauffolgenden Kalenderjahres wird die Partialoxidation erfindungsgemäß zweckmäßig wiederum wenigstens einmal unterbrochen, um das Gasgemisch G in erfindungsgemäßer Weise durch das Katalysatorfestbett 1 zu führen. Danach wird die Partialoxidation erfindungsgemäß vorteilhaft wie beschrieben wieder aufgenommen u.s.w..

In entsprechender Weise wird man bei der Ausübung des erfindungsgemäßen Verfahrens ein frisches Katalysatorfestbett 2 nach seiner Formierung normalerweise so betreiben, dass man nach Festlegung des Betriebs der ersten Reaktionsstufe und der Zusammensetzung des Reaktionsgasausgangsgemischs 2 und Festlegung der Belastung des Katalysatorfestbetts 2 mit Reaktionsgasausgangsgemisch 2 die Temperatur des Katalysatorfestbetts 2 (bzw. die Eintrittstemperatur des Temperiermediums in die Temperierzone des Rohrbündelreaktors) so einstellt, dass der Umsatz U^{Acr} des Acroleins bei einmaligem Durchgang des Reaktionsgasausgangsgemischs 2 durch das Katalysatorfestbett 2 wenigstens 90 mol.-% beträgt. Bei Verwendung günstiger Katalysatoren sind auch Werte für U^{Acr} ≥ 92 mol.-%, oder ≥ 94 mol.-%, oder ≥ 96 mol.-%, oder ≥ 98 mol.-%, und häufig sogar ≥ 99 mol.-% und mehr möglich.

Bei kontinuierlicher Ausübung der heterogen katalysierten Partialoxidation wird man die Zusammensetzung des Reaktionsgasausgangsgemischs 2 und die Belastung des Katalysatorfestbetts 2 mit Reaktionsgasausgangsgemisch 2 im wesentlichen konstant beibehalten (gegebenenfalls wird die Belastung an die fluktuierende Marktnachfrage angepasst). Einem Abfallen der Aktivität des Katalysatorfestbetts 2 über die Zeit wird man unter diesen Produktionsbedingungen normalerweise dadurch entgegenwirken, dass man von Zeit zu Zeit die Temperatur des Katalysatorfestbetts 2 (die Eintrittstemperatur des Temperiermediums in die Temperaturzone des Rohrbündelreaktors) erhöht (die Fließgeschwindigkeit des Temperiermediums wird normalerweise im wesentlichen ebenfalls beibehalten), um den Acroleinumsatz bei einmaligem Durchgang des Beschickungsgasgemischs im angestrebten Zielkorridor (d.h., bei Werten von ≥ 90 mol.-%, bzw. ≥ 92 mol.-%, bzw. ≥ 94 mol.-%, bzw. ≥ 96 mol.-%, oder ≥ 98 mol.-%, bzw. ≥ 99 mol.-%,) zu halten. Eine solche Vorgehensweise zieht jedoch die Eingangs dieser Schrift beschriebenen Nachteile mit sich.

Erfindungsgemäß vorteilhaft wird man deshalb so verfahren, dass man, bevor die vorgenommene Temperaturerhöhung des Katalysatorfestbetts 2 dauerhaft ≥ 10°C oder ≥ 8°C beträgt (bezogen auf die zuvor eingestellte Temperatur des Katalysatorfestbetts 2), die Gasphasenpartialoxidation wenigstens einmal unterbricht, um bei einer Temperatur des Katalysatorfestbetts 2 von 200 bis 450°C über das Katalysatorfestbett 1 das Gasgemisch G durch das Katalysatorfestbett 2 zu führen. Anschließend wird die Partialoxidation unter weitgehendem Beibehalt der Verfahrensbedingungen fortgesetzt (die Wiederherstellung der Acroleinbelastung des Katalysatorfestbetts 2 erfolgt vorzugsweise langsam, z.B. so wie in der DE-A 10337788 beschrieben) und die Temperatur des Katalysatorfestbetts 2 so eingestellt, dass der Acroleinumsatz den angestrebten Zielwert erreicht. In der Regel wird dieser Temperaturwert bei gleichem Umsatz bei einem etwas niedrigeren Wert liegen als die Temperatur, die das Katalysatorfestbett 2 vor der Unterbrechung der Partialoxidation und der erfindungsgemäßen Behandlung mit dem Gasgemisch G aufwies. Von diesem Temperaturwert des Katalysatorfestbetts 2 ausgehend wird die Partialoxidation unter weitgehendem Beibehalt der übrigen Bedingungen fortgesetzt und dabei dem Abfall der Aktivität des Katalysatorfestbetts 2 über die Zeit in zweckmäßiger Weise wiederum dadurch entgegengewirkt, dass man von Zeit zu Zeit die Temperatur des Katalysatorfestbetts 2 erhöht. Bevor die durchgeführte Temperaturerhöhung des Katalysatorfestbetts 2 dauerhaft ≥ 10°C oder ≥ 8°C beträgt, wird die Partialoxidation erfindungsgemäß wiederum wenigstens einmal unterbrochen, um das Gasgemisch G in erfindungsgemäßer Weise über das Katalysatorfestbett 1 durch das Katalysatorfestbett 2 zu führen. Danach wird die Partialoxidation erfindungsgemäß vorteilhaft wie beschrieben wieder aufgenommen u.s.w..

Es überrascht, dass im Langzeitbetrieb der erfindungsgemäßen zweistufigen heterogen katalysierten Gasphasenpartialoxidation von Propen zu Acrylsäure die Ausprägung der Heißpunktausdehnungen in beiden Reaktionsstufen ein günstigeres Verhalten zeigt, als bei den Verfahren gemäß des Standes der Technik.

Das erfindungsgemäße Verfahren ermöglicht so einerseits längere Standzeiten der Katalysatorfestbetten in den Reaktorsystemen, bevor diese teilweise oder vollständig ausgetauscht werden müssen. Auf der anderen Seite sind auch die über Zeit integral erzielten Reaktandenumsätze erhöht und die Selektivität der Zielproduktbildungen wird ebenfalls gefördert. Dazu trägt z.B. bei, dass der Ort des jeweiligen Heißpunktes beim erfindungsgemäßen Verfahren in beiden Reaktionsstufen entweder stationär bleibt oder in überraschender Weise normalerweise in Richtung der Eintrittsstelle des Beschickungsgasgemisches des jeweiligen Katalysatorfestbetts wandert. Damit wandert der Heißpunkt in der jeweiligen Reaktionsstufe im Reaktionsgasgemisch zunehmend in den Bereich vor, in dem der Acroleingehalt (1. Reaktionsstufe) bzw. Acrylsäuregehalt (2. Reaktionsstufe) noch wenig ausgeprägt ist. Dies mindert die Möglichkeit, dass bereits gebildetes Acrolein bzw. bereits gebildete Acrylsäure unter der Einwirkung der Heißpunkttemperatur partielle unerwünschte Vollverbrennung erleidet. Die Ermittlung der Heißpunkttemperatur kann beim erfindungsgemäßen Verfahren in Rohrbündelreaktoren z.B. mittels Thermorohren erfolgen, wie sie in der EP-A 873 783, der WO 03-076373 und in der EP-A 1 270 065 beschrieben sind. Die Anzahl solcher Thermorohre innerhalb eines Rohrbündelreaktors beträgt zweckmäßig 4 bis 20. Mit Vorteil befinden sie sich im Rohrbündelinneren gleichmäßig verteilt angeordnet.

Das erfindungsgemäße Verfahren ist insbesondere dann vorteilhaft, wenn es mit einer Belastung des Katalysatorfestbetts 1 mit Propen von ≥ 110 Nl/l·h, bzw. ≥ 120 Nl/l·h, bzw. ≥ 130 Nl/l·h betrieben wird. Die Acroleinbelastung des Katalysatorfestbetts 2 kann dann jeweils bis zu 20 Nl/l·h tiefer liegen.

Da das Gasgemisch G beim erfindungsgemäßen Verfahren das Katalysatorfestbett 2 erst nach Durchströmen des Katalysatorfestbetts 1 erreicht, enthält es zu Beginn der erfindungsgemäßen Durchströmung gegebenenfalls bis zu 5 Vol.-% CO. Es überrascht, dass sich dies nicht als nachteilig erwiesen hat. Dieser CO-Gehalt fällt innerhalb der Zeitdauer t_{G} normalerweise von seinem Ausgangswert A auf einen Wert E ab, der weniger als 50 %, bevorzugt weniger als 35 %, besonders bevorzugt weniger als 10 % oder weniger als 5 % des Ausgangswertes A beträgt. Ganz besonders bevorzugt fällt der CO-Gehalt C innerhalb t_{G} auf den Wert E=0 ab.

Vorgenannter Sachverhalt hat sich erfindungsgemäß als vorteilhaft erwiesen.

In gleicher Weise hat sich beim erfindungsgemäßen Verfahren ein geringfügiger Gehalt an Mo-Oxid-Hydrat (im ppm-Bereich) beim Eintritt des Gasgemischs G in das Katalysatorfestbett 2 als vorteilhaft erwiesen, den das Gasgemisch G in günstigen Fällen aus dem Katalysatorfestbett 1 oder aus Mo-Oxid Abscheidungen im Zwischenkühler austrägt.

Die Abtrennung der Acrylsäure aus dem Produktgasgemisch und damit einhergehende Kreisgasbildung kann wie in der WO 97/48669 beschrieben erfolgen.

Generell wird man die frisch beschickten Katalysatorfestbetten 1 und 2 so gestalten, dass, wie in der EP-A 990636 und EP-A 1106598 beschrieben, sowohl die Heißpunktausbildung als auch deren Temperatursensitivität möglichst gering sind. Ferner wird man sowohl bei der Erstinbetriebnahme als auch bei der Wiederinbetriebnahme nach Ausübung des erfindungsgemäßen Verfahrens die Belastung der Katalysatorfestbetten mit Reaktand vorteilhaft zunächst bei Werten ≤ 100 Nl/l·h belassen, bis sich ein stabiler Betrieb eingestellt hat.

### Beispiele

### A) Herstellung des in der ersten Reaktionsstufe verwendeten Katalysators

Herstellung eines ringförmigen Vollkatalysators mit der nachfolgenden Stöchiometrie des aktiven Multimetalloxids II:

[Bi₂W₂O₉ • 2WO₃]_{0,5} [MO₁₂CO_{5,5}Fe_{2,94}Si_{1,59}K_{0,06}Oₓ]₁.

### 1. Herstellung einer Ausgangsmasse 1

In 775 kg einer wäßrigen salpetersauren Wismutnitratlösung (11,2 Gew.-% Bi, freie Salpetersäure 3 bis 5 Gew.-%; Massendichte: 1,22 bis 1,27 g/ml) wurden bei 25°C portionsweise 209,3 kg Wolframsäure (72,94 Gew.-% W) eingerührt. Das resultierende wäßrige Gemisch wurde anschließend noch 2 h bei 25°C gerührt und anschließend sprühgetrocknet.

Die Sprühtrocknung erfolgte in einem Drehscheibensprühturm im Gegenstrom bei einer Gaseintrittstemperatur von 300 ± 10°C und einer Gasaustrittstemperatur von 100 ± 10°C. Das erhaltene Sprühpulver (Partikelgröße im wesentlichen einheitlich 30 µm), das einen Glühverlust von 12 Gew.-% aufwies (3 h bei 600°C unter Luft glühen), wurde anschließend mit 16,8 Gew.-% (bezogen auf das Pulver) Wasser in einem Kneter angeteigt und mittels eines Extruders (Drehmoment: ≤ 50 Nm) zu Strängen des Durchmessers 6 mm extrudiert. Diese wurden in Abschnitte von 6 cm geschnitten, auf einen 3-zonigen Bandtrockner bei einer Verweilzeit von 120 min bei Temperaturen von 90-95°C (Zone 1) und 125°C (Zone 2) und 125°C (Zone 3) an Luft getrocknet und dann bei einer Temperatur im Bereich von 780 bis 810°C thermisch behandelt (calciniert; im luftdurchströmten Drehrohrofen (1,54 m³ Innenvolumen, 200 Nm³ Luft/h)). Wesentlich bei der genauen Einstellung der Calcinationstemperatur ist, daß sie an der angestrebten Phasenzusammensetzung des Calcinationsprodukts orientiert zu erfolgen hat. Gewünscht sind die Phasen WO₃ (monoklin) und Bi₂W₂O₉, unerwünscht ist das Vorhandensein von γ-Bi₂WO₆ (Russellit). Sollte daher nach der Calcination die Verbindung γ-Bi₂WO₆ anhand eines Reflexes im Pulverröntgendiffraktogramm bei einem Reflexwinkel von 20 = 28,4° (CuKa-Strahlung) noch nachweisbar sein, so ist die Präparation zu wiederholen und die Calcinationstemperatur innerhalb des angegebenen Temperaturbereichs oder die Verweilzeit bei gleichbleibender Calcinationstemperatur zu erhöhen, bis das Verschwinden des Reflexes erreicht wird. Das so erhaltene vorgebildete calcinierte Mischoxid wurde gemahlen, so daß der X₅₀-Wert (vgl. Ullmann's Encyclopedia of Industrial Chemistry, 6th Edition (1998) Electronic Release, Kapitel 3.1.4 oder DIN 66141) der resultierenden Körnung 5 mm betrug. Das Mahlgut wurde dann mit 1 Gew.-% (bezogen auf das Mahlgut) feinteiligem SiO₂ der Fa. Degussa vom Typ Si-pernat^{®} (Rüttelgewicht 150 g/l; X₅₀-Wert der SiO₂-Partikel betrug 10 µm, die BET-Oberfläche betrug 100 m²/g) vermischt.

### 2. Herstellung einer Ausgangsmasse 2

Eine Lösung A wurde hergestellt, indem man bei 60°C unter Rühren in 600 I Wasser 213 kg Ammoniumheptamolybdattetrahydrat (81,5 Gew.-% MoO₃) löste und die resultierende Lösung unter Aufrechterhaltung der 60°C und Rühren mit 0,97 kg einer 20°C aufweisenden wässrigen Kaliumhydroxidlösung (46,8 Gew.-% KOH) versetzte.

Eine Lösung B wurde hergestellt indem man bei 60°C in 262,9 kg einer wässrigen Co-(II)-baltnitratlösung (12,4 Gew.-% Co) 116,25 kg einer wäßrigen Eisen-(III)-nitratlösung (14,2 Gew.-% Fe) eintrug. Anschließend wurde unter Aufrechterhaltung der 60°C die Lösung B über einen Zeitraum von 30 Minuten kontinuierlich in die vorgelegte Lösung A gepumpt. Anschließend wurde 15 Minuten bei 60°C gerührt. Dann wurden dem resultierenden wäßrigen Gemisch 19,16 kg eines Kieselgels der Fa. Dupont vom Typ Ludox (46,80 Gew.-% SiO₂, Dichte: 1,36 bis 1,42 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Gegenstrom sprühgetrocknet (Gaseintrittstemperatur: 400 ± 10°C, Gasaustrittstemperatur: 140 ± 5°C). Das resultierende Sprühpulver wies einen Glühverlust von ca. 30 Gew.-% (3 h bei 600°C unter Luft glühen) und eine im wesentlichen einheitliche Körnung von 30 µm auf.

### Herstellung der Multimetalloxidaktivmasse II

Die Ausgangsmasse 1 wurde mit der Ausgangsmasse 2 in den für eine Multimetalloxidaktivmasse der Stöchiometrie

[Bi₂W₂O₉·2WO₃]_{0,5}[Mo₁₂Co_{5,5}Fe_{2,94}Si_{1,59}K_{0,08}Oₓ]₁

erforderlichen Mengen in einem Mischer mit Messerköpfen homogen vermischt. Bezogen auf die vorgenannte Gesamtmasse wurden zusätzlich 1 Gew.-% feinteiliges Graphit der Fa. Timcal AG (San Antonio, US), vom Typ TIMREX P44 (Siebanalyse: min. 50 Gew.-% < 24 mm, max. 10 Gew.-% ≥ 24 µm und ≤ 48 µm, max. 5 Gew.-% > 48 µm, BET-Oberfläche: 6 bis 13 m²/g) homogen eingemischt. Das resultierende Gemisch wurden dann in einem Kompaktor (Fa. Hosokawa Bepex GmbH, D-74211 Leingarten) vom Typ Kompaktor K200/100 mit konkaven, geriffelten Glattwalzen gefahren (Spaltweite: 2,8 mm, Siebweite: 1,0 mm, Siebweite Unterkorn: 400 µm, Presssollkraft: 60 kN, Schneckendrehzahl: 65 bis 70 Umdrehungen je Minute). Das resultierende Kompaktat wies eine Härte von 10 N und eine im wesentlichen einheitliche Körnung von 400 µm bis 1 mm auf.

Das Kompaktat wurde anschließend mit, bezogen auf sein Gewicht, weiteren 2 Gew.-% desselben Graphit vermischt und anschließend in einem Kilian Rundläufer (Tablettiermaschine) vom Typ R x 73, der Fa. Kilian, D-50735 Köln, unter Stickstoffatmosphäre zu ringförmigen Vollkatalysatorvorläuferformkörpern der Geometrie (Außendurchmesser x Länge x Innendurchmesser) 5 mm x 3 mm x 2 mm mit einer Seitendruckfestigkeit von 19 N ± 3 N verdichtet.

Als Seitendruckfestigkeit wird dabei in dieser Schrift die Druckfestigkeit bei Stauchung des ringförmigen Vollkatalysatorvorläuferformkörpers senkrecht zur Zylinderfläche (d.h., parallel zur Fläche der Ringöffnung) verstanden.

Dabei beziehen sich alle Seitendruckfestigkeiten dieser Schrift auf eine Bestimmung mittels einer Material-Prüfmaschine der Firma Zwick GmbH & Co. (D-89079 Ulm) des Typs Z 2.5 / TS1 S. Diese Material-Prüfmaschine ist für quasistatische Beanspruchung mit zügigem, ruhendem, schwellendem oder wechselndem Verlauf konzipiert. Sie ist für Zug-, Druck- und Biegeversuche geeignet. Der installierte Kraftaufnehmer des Typs KAF-TC der Firma A.S.T. (D-01307 Dresden) mit der Herstellnummer 03-2038 wurde dabei entsprechend der DIN EN ISO 7500-1 kalibriert und war für den Messbereich 1-500N einsetzbar (relative Messunsicherheit: ± 0,2 %).

Die Messungen wurden mit folgenden Parametern durchgeführt:
Vorkraft: 0,5 N.
Vorkraft-Geschwindigkeit: 10 mm/min.
Prüfgeschwindigkeit: 1,6 mm/min.

Dabei wurde der obere Stempel zunächst langsam bis kurz vor Zylinderfläche des ringförmigen Vollkatalysatorvorläuferformkörpers abgesenkt. Dann wurde der obere Stempel abgestoppt, um anschließend mit der deutlich langsameren Prüfgeschwindigkeit mit minimaler, zu weiterer Absenkung erforderlicher, Vorkraft abgesenkt zu werden.

Die Vorkraft, bei der der Vollkatalysatorvorläuferformkörper Rissbildung zeigt, ist die Seitendruckfestigkeit (SDF).

Zur abschließenden thermischen Behandlung wurden jeweils 1000 g der Vollkatalysatorvorläuferformkörper in einem Luft durchströmten Muffelofen (60 l Innenvolumen, 1 l/h Luft pro Gramm Vollkatalysatorvorläuferformkörper) zunächst mit einer Aufheizrate von 180°C/h von Raumtemperatur (25°C) auf 190°C aufgeheizt. Diese Temperatur wurde für 1 h aufrechterhalten und dann mit einer Aufheizrate von 60°C/h auf 210°C erhöht. Die 210°C wurden wiederum während 1 h aufrechterhalten, bevor sie mit einer Aufheizrate von 60°C/h, auf 230°C erhöht wurden. Diese Temperatur wurde ebenfalls 1 h aufrechterhalten, bevor sie, wiederum mit einer Aufheizrate von 60°C/h, auf 265°C erhöht wurde. Die 265°C wurden anschließend ebenfalls während 1 h aufrechterhalten. Danach wurde zunächst auf Raumtemperatur abgekühlt und damit die Zersetzungsphase im wesentlichen abgeschlossen. Dann wurde mit einer Aufheizrate von 180°C/h auf 465°C erhitzt und diese Calcinationstemperatur während 4 h aufrechterhalten.

Dabei wurden aus den ringförmigen Vollkatalysatorvorläuferformkörpern ringförmige Vollkatalysatoren erhalten.

Die spezifische Oberfläche O, das Porengesamtvolumen V, der Porendurchmesser d^{max} der den größten Beitrag zum Porengesamtvolumen leistet, sowie die prozentualen Anteile derjenigen Porendurchmesser am Porengesamtvolumen, deren Durchmesser > 0,1 und ≤ 1 µm betragen, lautete für die erhaltenen ringförmigen Vollkatalysatoren wie folgt:
O = 7,6 cm²/g.
V = 0,27 cm³/g.
d^{max} [µm] = 0,6.
V^{0,1}₁-% = 79.

Außerdem betrug das Verhältnis R von scheinbarer Massendichte zu wahrer Massendichte p (so wie es in der EP-A 1340538 definiert ist) 0,66.

In großtechnischer Menge wurde der selbe ringförmige Katalysator durch thermische Behandlung wie in Beispiel 1 der DE-A 10046957 (die Schütthöhe in der Zersetzung (Kammern 1 bis 4) belief sich dabei jedoch vorteilhaft auf 44 mm bei einer Verweilzeit pro Kammer von 1,46 h und in der Calcination (Kammern 5 bis 8) belief sie sich vorteilhaft auf 130 mm bei einer Verweilzeit von 4,67 h) beschrieben mittels einer Bandcalziniervorrichtung hergestellt; die Kammern besaßen eine Grundfläche (bei einer einheitlichen Kammerlänge von 1,40 m) von 1,29 m² (Zersetzung) und 1,40 m² (Calcination) und wurden von unten durch das grobmaschige Band von 75 Nm³/h Zuluft durchströmt, die mittels rotierender Ventilatoren angesaugt wurden. Innerhalb der Kammern war die zeitliche und örtliche Abweichung der Temperatur vom Sollwert stets ≤ 2°C. Im übrigen wurde wie in Beispiel 1 der DE-A 10046957 beschrieben verfahren.

### B) Herstellung des in der zweiten Reaktionsstufe verwendeten Katalysators

### 1. Allgemeine Beschreibung des zur Calcination im Rahmen der Herstellung von Ausgangsmassen 1 und 2 verwendeten Drehrohrofens

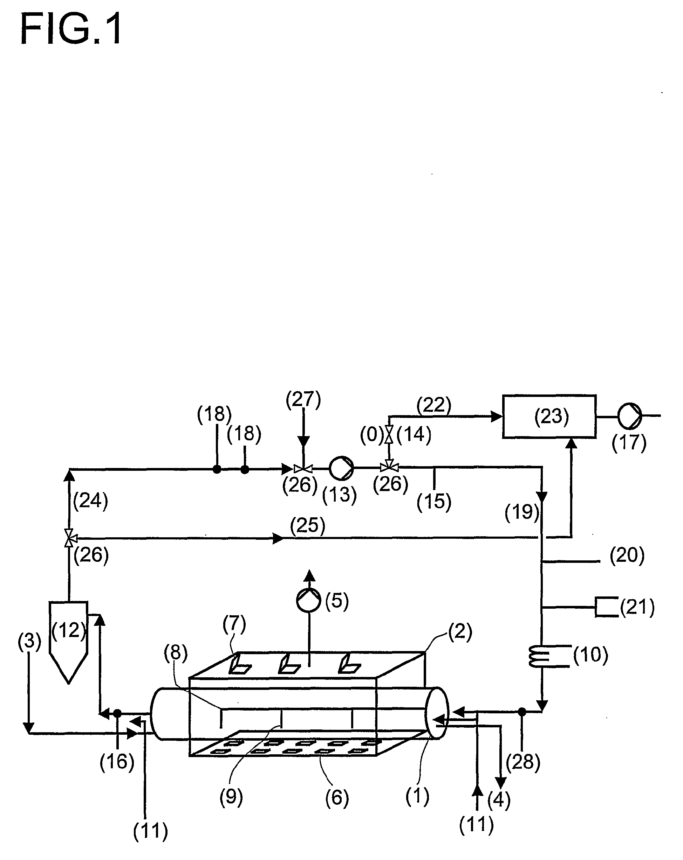

Eine schematische Darstellung des Drehrohrofens zeigt die dieser Schrift beiliegende Figur 1. Die nachfolgenden Bezugsziffern beziehen sich auf diese Figur 1.

Zentrales Element des Drehrohrofens ist das Drehrohr (1). Es ist 4000 mm lang und weist einen Innendurchmesser von 700 mm auf. Es ist aus Edelstahl 1.4893 gefertigt und weist eine Wanddicke von 10 mm auf.

Auf der Innenwand des Drehrohrofens sind Hublanzen angebracht, die eine Höhe von 5 cm und eine Länge von 23,5 cm aufweisen. Sie dienen primär dem Zweck, das thermisch im Drehrohrofen zu behandelnde Gut anzuheben und dadurch zu durchmischen.

Auf ein und derselben Höhe des Drehrohrofens sind um den Umfang äquidistant (jeweils 90° Abstand) jeweils vier Hublanzen angebracht (ein Quadrupel). Längs des Drehrohrofens befinden sich acht solcher Quadrupel (jeweils im Abstand von 23,5 cm). Die Hublanzen zweier benachbarter Quadrupel sitzen am Umfang gegeneinander versetzt. Am Anfang und am Ende Drehrohrofens (erste und letzte 23,5 cm) befinden sich keine Hublanzen.

Das Drehrohr rotiert frei in einem Quader (2), der vier in der Länge des Drehrohres aufeinanderfolgende, gleich lange, elektrisch beheizte (Widerstandsheizung) Heizzonen aufweist, von denen jede den Umfang des Drehrohrofens umschließt. Jede der Heizzonen kann den entsprechenden Drehrohrabschnitt auf Temperaturen zwischen Raumtemperatur und 850°C erhitzen. Die maximale Heizleistung jeder Heizzone beträgt 30 kW. Der Abstand zwischen elektrischer Heizzone und Drehrohraußenfläche beträgt etwa 10 cm. Am Anfang und am Ende ragt das Drehrohr ca. 30 cm aus dem Quader heraus.

Die Umdrehungsgeschwindigkeit kann zwischen 0 und 3 Umdrehungen pro Minute variabel eingestellt werden. Das Drehrohr ist sowohl links- als auch rechts-drehbar. Bei Rechtsdrehung verweilt das Materialgut im Drehrohr, bei Linksdrehung wird das Materialgut vom Eintrag (3) zum Austrag (4) gefördert. Der Neigungswinkel des Drehrohres zur Horizontalen kann zwischen 0° und 2° variabel eingestellt werden. Im diskontinuierlichen Betrieb liegt er faktisch bei 0°. Im kontinuierlichen Betrieb befindet sich die tiefstliegendste Stelle des Drehrohrs beim Materialgutaustrag. Eine Schnellkühlung des Drehrohres kann dadurch erfolgen, dass die elektrischen Heizzonen abgeschaltet und ein Ventilator (5) eingeschaltet wird. Dieser saugt durch im unteren Boden des Quaders befindliche Löcher (6) Umgebungsluft an, und fördert sie durch drei im Deckel befindliche Klappen (7) mit variabel einstellbarer Öffnung.

Der Materialguteintrag wird über eine Zellenradschleuse kontrolliert (Massenkontrolle). Der Materialgutaustrag wird, wie bereits erwähnt, über die Drehrichtung des Drehrohrs gesteuert.

Bei diskontinuierlichem Betrieb des Drehrohres kann eine Materialgutmenge von 250 bis 500 kg thermisch behandelt werden. Sie befindet sich dabei normalerweise ausschließlich im beheizten Teil des Drehrohres.

Von einer auf der zentralen Achse des Drehrohres liegenden Lanze (8) führen in Abständen von 800 mm insgesamt drei Thermoelemente (9) vertikal ins Materialgut. Sie ermöglichen die Bestimmung der Temperatur des Materialguts. In dieser Schrift wird unter Temperatur des Materialgutes das arithmetische Mittel der drei Thermoelementtemperaturen verstanden. Innerhalb des im Drehrohr befindlichen Materialguts beträgt die maximale Abweichung zweier gemessener Temperaturen erfindungsgemäß zweckmäßig weniger als 30°C, bevorzugt weniger als 20°C, besonders bevorzugt weniger als 10°C und ganz besonders bevorzugt weniger als 5 bzw. 3°C.

Durch das Drehrohr können Gasströme geführt werden, mittels derer sich die Calcinationsatmosphäre bzw. allgemein die Atmosphäre der thermischen Behandlung des Materialgutes einstellen lässt.

Der Erhitzer (10) bietet die Möglichkeit, den ins Drehrohr geführten Gasstrom vorab seines Eintritts ins Drehrohr auf die gewünschte Temperatur zu erwärmen (z.B. auf die für das Materialgut im Drehrohr gewünschte Temperatur). Die maximale Leistung des Erhitzers liegt bei 1 x 50 kW + 1 x 30 kW. Vom Prinzip her kann es sich beim Erhitzer (10) z.B. um einen indirekten Wärmetauscher handeln. Solch ein Erhitzer kann prinzipiell auch als Kühler genutzt werden. In der Regel handelt es sich aber um einen Elektroerhitzer, bei dem der Gasstrom über Strom beheizte Metalldrähte geführt wird (zweckmäßigerweise ein CSN Durchlauferhitzer, Typ 97D/80 der Fa. C. Schniewindt KG, 58805 Neuerade - DE).

Prinzipiell sieht die Drehrohrvorrichtung die Möglichkeit vor, den durch das Drehrohr geführten Gasstrom teilweise oder vollständig im Kreis zu führen. Die dazu erforderliche Kreisleitung wird am Drehrohreintritt und am Drehrohraustritt über Kugellager oder über Graphitpressdichtungen mit dem Drehrohr beweglich verbunden. Diese Verbindungen werden mit Inertgas (z.B. Stickstoff) gespült (Sperrgas). Die beiden Spülströme (11) ergänzen den durch das Drehrohr geführten Gasstrom am Eintritt ins Drehrohr und am Austritt aus dem Drehrohr. Zweckmäßigerweise verjüngt sich dabei das Drehrohr an seinem Anfang und an seinem Ende und ragt in das zu- bzw. wegführende Rohr der Kreisleitung hinein.

Hinter dem Austritt des durch das Drehrohr geführten Gasstroms befindet sich ein Zyklon (12), zur Abtrennung von mit dem Gasstrom mitgerissenen Feststoffpartikeln (der Zentrifugalabscheider trennt in der Gasphase suspendierte Feststoffpartikel durch Zusammenwirken von Zentrifugal- und Schwerkraft ab; die Zentrifugalkraft des als Spiralwirbel rotierenden Gasstroms beschleunigt die Sedimentation der suspendierten Teilchen).

Die Förderung des Kreisgasstroms (24) (die Gaszirkulation) erfolgt mittels eines Kreisgasverdichters (13) (Ventilators), der in Richtung des Zyklons ansaugt und in die andere Richtung drückt. Unmittelbar hinter dem Kreisgasverdichter liegt der Gasdruck in der Regel oberhalb einer Atmosphäre. Hinter dem Kreisgasverdichter befindet sich ein Kreisgasauslass (über ein Regelventil (14) kann Kreisgas ausgeschleust werden). Eine hinter dem Auslass befindliche Blende (Querschnittsverjüngung um etwa einen Faktor 3, Druckminderer) (15) erleichtert den Auslass.

Über das Regelventil lässt sich der Druck hinter dem Drehrohrausgang regulieren. Dies geschieht im Zusammenspiel mit einem hinter dem Drehrohrausgang angebrachten Drucksensor (16), dem Abgasverdichter (17) (Ventilator), der zum Regelventil hin gerichtet ansaugt, dem Kreisgasverdichter (13) und der Frischgaszufuhr. Relativ zum Außendruck kann der Druck (unmittelbar) hinter dem Drehrohrausgang z.B. bis zu +1,0 mbar oberhalb und z.B. bis zu -1,2 mbar unterhalb liegend eingestellt werden. D.h., der Druck des das Drehrohr durchströmenden Gasstroms kann beim Verlassen des Drehrohrs unterhalb des Umgebungsdrucks des Drehrohrs liegen.

Ist beabsichtigt, den durch das Drehrohr geführten Gasstrom auch nicht wenigstens anteilig im Kreis zu führen, wird die Verbindung zwischen Zyklon (12) und Kreisgasverdichter (13) nach dem Dreiwegehahnprinzip (26) geschlossen und der Gasstrom auf direktem Weg in die Abgasreinigungsvorrichtung (23) geführt. Die hinter dem Kreisgasverdichter befindliche Verbindung zur Abgasreinigungsvorrichtung wird in diesem Fall ebenfalls nach dem Dreiwegehahnprinzip geschlossen. Besteht der Gasstrom im wesentlichen aus Luft, wird diese in diesem Fall über den Kreisgasverdichter (13) angesaugt (27). Die Verbindung zum Zyklon ist nach dem Dreiwegehahnprinzip geschlossen. Vorzugsweise wird der Gasstrom in diesem Fall durch das Drehrohr gesaugt, so dass der Drehrohrinnendruck kleiner als der Umgebungsdruck ist.

Bei kontinuierlichem Betrieb der Drehrohrofenvorrichtung wird der Druck hinter dem Drehrohrausgang vorteilhaft -0,2 mbar unterhalb des Außendruckes liegend eingestellt. Bei diskontinuierlichem Betrieb der Drehrohrvorrichtung wird der Druck hinter dem Drehrohrausgang vorteilhaft -0,8 mbar unterhalb des Außendruckes liegend eingestellt. Der leichte Unterdruck dient dem Zweck einer Vermeidung einer Kontamination der Umgebungsluft mit Gasgemisch aus dem Drehrohrofen.

Zwischen dem Kreisgasverdichter und dem Zyklon befinden sich Sensoren (18), die zum Beispiel den Ammoniakgehalt und den Sauerstoffgehalt im Kreisgas bestimmen. Der Ammoniaksensor arbeitet bevorzugt nach einem optischen Messprinzip (die Absorption von Licht bestimmter Wellenlänge korreliert proportional zum Ammoniakgehalt des Gases) und ist zweckmäßig ein Gerät der Fa. Perkin & Elmer vom Typ MCS 100. Der Sauerstoffsensor fußt auf den paramagnetischen Eigenschaften des Sauerstoffs und ist zweckmäßigerweise ein Oximat der Fa. Siemens vom Typ Oxymat MAT SF 7MB1010-2CA01-1 AA1-Z.

Zwischen der Blende (15) und dem Erhitzer (10) können Gase wie Luft, Stickstoff, Ammoniak oder andere Gase zum tatsächlich rezirkulierten Kreisgasanteil (19) zudosiert werden. Häufig wird eine Grundlast an Stickstoff zudosiert (20). Mit einem separaten Stickstoff/Luft Splitter (21) kann auf den Messwert des Sauerstoffsensors reagiert werden.

Der ausgeschleuste Kreisgasanteil (22) (Abgas) enthält häufig nicht völlig unbedenkliche Gase wie NOₓ, Essigsäure, NH₃, u.s.w.), weshalb diese im Normalfall in einer Abgasreinigungsvorrichtung abgetrennt werden (23).

Dazu wird das Abgas in der Regel zunächst über eine Waschkolonne geführt (ist im wesentlichen eine an Einbauten freie Kolonne, die vor ihrem Ausgang eine trennwirksame Packung enthält; das Abgas und wässriger Sprühnebel werden im Gleichstrom und im Gegenstrom geführt (2 Sprühdüsen mit entgegengesetzter Sprührichtung).

Aus der Waschkolonne kommend wird das Abgas in eine Vorrichtung geführt, die einen Feinstaubfilter (in der Regel ein Bündel aus Schlauchfiltern) enthält aus dessen Innerem das eingedrungene Abgas weggeführt wird. Dann wird abschließend in einer Muffel verbrannt.

Mittels eines Sensors (28) der Fa. KURZ Instruments, Inc., Montery (USA) vom Typ Modell 455 Jr wird die Menge des vom Sperrgas verschiedenen, dem Drehrohr zugeführten, Gasstroms gemessen und geregelt (Messprinzip: thermalkonvektive Massendurchflussmessung unter Anwendung eines Gleichtemperatur-Anemometers).

Bei kontinuierlichem Betrieb werden Materialgut und Gasphase im Gegenstrom durch den Drehrohrofen geführt.

Stickstoff bedeutet im Zusammenhang mit diesem Beispiel immer Stickstoff mit einer Reinheit > 99 Vol.-%.

### 2. Herstellung der Ausgangsmasse 1 (Phase B) mit der Stöchiometrie Cu₁Mo_{0,5}W_{0,5}O₄

Zu 603 I Wasser wurden 98 I einer 25 gew.-%igen wässrigen NH₃-Lösung gegeben. Anschließend wurden in der wässrigen Mischung 100 kg Kupfer(II)acetathydrat (Gehalt: 40,0 Gew.-% CuO) gelöst, wobei eine klare, tiefblaue wässrige Lösung 1 entstand, die 3,9 Gew.-% Cu enthielt und Raumtemperatur aufwies.

Unabhängig von der Lösung 1 wurden 620 I Wasser auf 40°C erwärmt. Darin wurden unter Aufrechterhaltung der 40°C 27,4 kg Ammoniumheptamolybdattetrahydrat (81,5 Gew.-% MoO₃) innerhalb von 20 min. unter Rühren gelöst. Dann wurden 40,4 kg Ammoniumparawolframatheptahydrat (88,9 Gew.-% WO₃) zugefahren und nach Aufheizen auf 90°C innerhalb von 45 min. bei dieser Temperatur unter Rühren gelöst. Es wurde eine klare, gelb-orange gefärbte wässrige Lösung 2 erhalten.

Anschließend wurde die wässrige Lösung 1 in die 90°C aufweisende Lösung 2 eingerührt, wobei die Temperatur der Gesamtmischung nicht unter 80°C sank. Die resultierende wässrige Suspension wurde 30 min. bei 80°C nachgerührt. Danach wurde sie mit einem Sprühtrockner der Fa. Niro-Atomizer (Kopenhagen) vom Typ S-50-N/R sprühgetrocknet (Gaseintrittstemperatur: 315°C, Gasaustrittstemperatur: 110°C, Gleichstrom). Das Sprühpulver wies einen Partikeldurchmesser von 2 bis 50 µm auf.

100 kg von so erhaltenem hellgrünem Sprühpulver wurden in einen Kneter der Fa. AMK (Aachener Misch- und Knetmaschinen Fabrik) vom Typ VIU 160 (Sigma Schaufeln) dosiert und unter Zugabe von 8 I Wasser geknetet (Verweilzeit: 30 min, Temperatur: 40 bis 50°C). Danach wurde das Knetgut in einen Extruder entleert und mittels des Extruders (Fa. Bonnot Company (Ohio), Typ: G 103-10/D7A-572K (6" Extruder W/Packer) zu Strängen (Länge: 1-10 cm; Durchmesser: 6 mm) geformt. Auf einem Bandtrockner wurden die Stränge 1 h bei einer Temperatur von 120°C (Materialguttemperatur) getrocknet. Die getrockneten Stränglinge wurden anschließend im unter "1." beschriebenen Drehrohrofen wie folgt thermisch behandelt (calciniert):
- die thermische Behandlung erfolgte kontinuierlich mit einem Materialguteintrag von 50 kg/h an Stränglingen;
- der Neigungswinkel des Drehrohres zur Horizontalen betrug 2°;
- im Gegenstrom zum Materialgut wurde ein Luftstrom von 75 Nm³/h durch das Drehrohr geführt, der von insgesamt (2 x 25) 50 Nm³/h Sperrgas der Temperatur 25°C ergänzt wurde;
- der Druck hinter dem Drehrohrausgang lag 0,8 mbar unter dem Außendruck;
- das Drehrohr rotierte mit 1,5 Umdrehungen/min links herum;
- es wurde keine Kreisgasfahrweise angewendet;
- beim ersten Durchgang der Stränglinge durch das Drehrohr wurde die Temperatur der Drehrohraußenwand auf 340°C eingestellt, der Luftstrom wurde mit einer Temperatur von 20 bis 30°C in das Drehrohr geführt;
- anschließend wurden die Stränglinge mit der selben Durchsatzmenge und abgesehen von den nachfolgenden Unterschieden unter den gleichen Bedingungen durch das Drehrohr geführt:
- die Temperatur der Drehrohrwand wurde auf 790°C eingestellt;
- der Luftstrom wurde auf eine Temperatur von 400°C erwärmt in das Drehrohr geführt.

Anschließend wurden die eine rotbraune Farbe aufweisenden Stränglinge auf einer Biplexquerstromsichtmühle (BQ 500) der Fa. Hosokawa-Alpine (Augsburg) auf einen mittleren Partikeldurchmesser von 3 bis 5 µm gemahlen. Die so erhaltene Ausgangsmasse 1 wies eine BET-Oberfläche ≤ 1 m²/g auf. Mittels Röntgenbeugung wurden folgende Phasen festgestellt:
1. CuMoO₄-III mit Wolframitstruktur;
2. HT-Kupfermolybdat.

### 3. Herstellung der Ausgangsmasse 2 (Phase C) mit der Stöchiometrie CuSb₂O₆

Unter Rühren wurden 52 kg Antimontrioxid (99,9 Gew.-% Sb₂O₃) in 216 I Wasser (25°C) suspendiert. Die resultierende wässrige Suspension wurde auf 80°C erwärmt. Anschließend wurde unter Aufrechterhaltung der 80°C 20 min nachgerührt. Anschließend wurden innerhalb einer Stunde 40 kg einer 30 gew.-%igen wässrigen Wasserstoffperoxidlösung zugegeben, wobei die 80°C aufrechterhalten wurden. Unter Beibehalt dieser Temperatur wurde 1,5 Stunden nachgerührt. Dann wurden 20 I 60°C warmes Wasser zugegeben und so eine wässrige Suspension 1 erhalten. In diese wurden bei einer Temperatur von 70°C 618,3 kg einer wässrigen ammoniakalischen Kupfer(II)acetatlösung (60,8 g Kupferacetat pro kg Lösung und 75 I einer 25 gew.-%igen wässrigen Ammoniaklösung in den 618,3 kg Lösung) eingerührt. Dann wurde auf 95°C erwärmt und bei dieser Temperatur 30 min nachgerührt. Dann wurden noch 50 I 70°C warmes Wasser ergänzt und auf 80°C erwärmt.

Schließlich wurde die wässrige Suspension sprühgetrocknet (Sprühtrockner der Fa. Niro-Atomizer (Kopenhagen) vom Typ S-5O-N/R, Gaseintrittstemperatur 360°C, Gasaustrittstemperatur 110°C, Gleichstrom). Das Sprühpulver wies einen Partikeldurchmesser von 2 bis 50 µm auf.

75 kg von so erhaltenem Sprühpulver wurden in einem Kneter der Fa. AMK (Aachener Misch- und Knetmaschinen Fabrik vom Typ VIU 160 (Sigma Schaufeln) dosiert und unter Zugaben von 12 I Wasser geknetet (Verweilzeit: 30 min, Temperatur 40 bis 50°C). Danach wurde das Knetgut in einen Extruder (gleicher Extruder wie bei Phase B Herstellung) entleert und mittels des Extruders zu Strängen (Länge 1-10 cm; Durchmesser 6 mm) geformt. Auf einem Bandtrockner wurden die Stränge 1 h bei einer Temperatur von 120°C (Materialguttemperatur) getrocknet.

250 kg von so erhaltenen Strängen wurden im unter "1." beschriebenen Drehrohrofen wie folgt thermisch behandelt (calciniert):
- die thermische Behandlung erfolgte diskontinuierlich mit einer Materialgutmenge von 250 kg;
- der Neigungswinkel des Drehrohres zur Horizontalen betrug ≈ 0°;
- das Drehrohr rotierte mit 1,5 Umdrehungen/min rechts herum;
- durch das Drehrohr wurde ein Gasstrom von 205 Nm³/h geführt; dieser bestand zu Beginn der thermischen Behandlung aus 180 Nm³/h Luft und 1 x 25 Nm³/h N₂ als Sperrgas; der das Drehrohr verlassende Gasstrom wurde um weitere 1 x 25 Nm³/h N₂ ergänzt; von diesem Gesamtstrom wurden 22 - 25 Vol.-% ins Drehrohr rückgeführt und der Rest ausgelassen; die Auslassmenge wurde durch das Sperrgas und als Restmenge durch Frischluft ergänzt;
- der Gasstrom wurde mit 25°C ins Drehrohr geführt;
- der Druck hinter dem Drehrohrausgangs lag 0,5 mbar unterhalb Außendruck (Normaldruck);
- die Temperatur im Materialgut wurde zunächst innerhalb von 1,5 h linear von 25°C auf 250°C erhöht; dann wurde die Temperatur im Materialgut innerhalb von 2 h linear von 250°C auf 300°C erhöht und diese Temperatur 2 h gehalten; dann wurde die Temperatur im Materialgut innerhalb von 3 h linear von 300°C auf 405°C erhöht und diese Temperatur anschließend 2 h gehalten; dann wurden die Heizzonen abgeschaltet und die Temperatur innerhalb des Materialgutes durch Aktivierung der Schnellkühlung des Drehrohres durch Ansaugen von Luft innerhalb von 1 h auf eine unterhalb von 100°C liegende Temperatur erniedrigt und schließlich auf Umgebungstemperatur abgekühlt.

Die so resultierende pulverförmige Ausgangsmasse 2 wies eine spezifische BET-Oberfläche von 0,6 m²/g und die Zusammensetzung CuSb₂O₆ auf. Das Pulver-Röntgendiagramm des erhaltenen Pulvers zeigte im wesentlichen die Beugungsreflexe von CuSb₂O₆ (Vergleichsspektrum 17-0284 der JCPDS-ICDD-Kartei).

### 4. Herstellung der Ausgangsmasse 3 mit der Stöchiometrie Mo₁₂V_{3,35}W_{1,38}

In einem Rührkessel wurden bei 25°C unter Rühren 900 I Wasser vorgelegt. Anschließend wurden 122,4 kg Ammoniumheptamolybdattetrahydrat (81,5 Gew.-% MoO₃) zugegeben und unter Rühren auf 90°C erhitzt. Dann wurden unter Aufrechterhaltung der 90°C zunächst 22,7 kg Ammoniummetavanadat und schließlich 20,0 kg Ammoniumparawolframatheptahydrat (88,9 Gew.-% WO₃) eingerührt. Durch insgesamt 80-minütiges Rühren bei 90°C wurde eine klare orangefarbene Lösung erhalten. Diese wurde auf 80°C abgekühlt. Dann wurden unter Beibehalt der 80°C zunächst 18,8 kg Essigsäure (= 100 gew.-%ig, Eisessig) und dann 24 l 25 gew.-%ige wässrige Ammoniaklösung eingerührt.

Die Lösung blieb klar und orangefarben und wurde mit einem Sprühtrockner der Fa. Niro-Atomizer (Kopenhagen) vom Typ S-50-N/R sprühgetrocknet (Gaseintrittstemperatur: 260°C, Gasaustrittstemperatur: 110°C, Gleichstrom). Das resultierende Sprühpulver bildete die Ausgangsmasse 3 und wies einen Partikeldurchmesser von 2 bis 50 µm auf.

### 5. Herstellung der thermisch zu behandelnden Trockenmasse mit der Stöchiometrie (Mo₁₂V_{3,46}W_{1,39})_{0,87} (CuMo_{0,5}W_{0,5}O₄)_{0,4} (CuSb₂O₆)_{0,4}

In einem Trogkneter der Fa. AMK (Aachener Misch- und Knetmaschinen Fabrik) vom Typ VIU 160 mit zwei Sigmaschaufeln wurden 75 kg Ausgangsmasse 3, 5,2 I Wasser und 6,9 kg Essigsäure (100 Gew.-% Eisessig) vorgelegt und während 22 min geknetet. Anschließend wurden 3,1 kg Ausgangsmasse 1 und 4,7 kg Ausgangsmasse 2 zugegeben und während weiterer 8 min geknetet (T = 40 bis 50°C).

Danach wurde das Knetgut in einen Extruder (gleicher Extruder wie bei Phase B Herstellung) entleert und mittels des Extruders zu Strängen (1 bis 10 cm Länge, 6 mm Durchmesser) geformt. Diese wurden auf einem Bandtrockner während 1 h bei einer Temperatur (Materialguttemperatur) von 120°C getrocknet.

306 kg der getrockneten Stränglinge wurden anschließend im unter "1." beschriebenen Drehrohrofen wie folgt thermisch behandelt:
- die thermische Behandlung erfolgte diskontinuierlich mit einer Materialgutmenge von 306 kg;
- der Neigungswinkel des Drehrohres zur Horizontalen betrug ≈ 0°;
- das Drehrohr rotierte mit 1,5 Umdrehungen/min rechts herum;
- zunächst wurde die Materialguttemperatur innerhalb von 2 h im wesentlichen linear von 25°C auf 100°C erhöht; während dieser Zeit wird durch das Drehrohr ein (im wesentlichen) Stickstoffstrom von 205 Nm³/h zugeführt. Im stationären Zustand (nach Verdrängung der ursprünglich enthaltenen Luft) ist dieser wie folgt zusammengesetzt:
   110 Nm³/h Grundlast - Stickstoff (20),
   25 Nm³/h Sperrgas - Stickstoff (11), und
   70 Nm³/h rezirkuliertes Kreisgas (19).
   Der Sperrgas-Stickstoff wurde mit einer Temperatur von 25°C zugeführt. Das Gemisch der beiden anderen Stickstoffströme wurde jeweils mit der Temperatur ins Drehrohr geführt, die das Materialgut im Drehrohr jeweils aufwies.
- anschließend wurde die Materialguttemperatur mit einer Heizrate von 0,7°C/min von 100°C auf 320°C aufgeheizt;
   bis zum Erreichen einer Materialguttemperatur von 300°C wurde durch das Drehrohr ein Gasstrom von 205 Nm³/h geführt, der wie folgt zusammengesetzt war:
   110 Nm³/h bestehend aus Grundlast - Stickstoff (20) und im Drehrohr freigesetzten Gasen,
   25 Nm³/h Sperrgas - Stickstoff (11) und
   70 Nm³/h rezirkuliertes Kreisgas (19).
   Der Sperrgas-Stickstoff wurde mit einer Temperatur von 25°C zugeführt. Das Gemisch der beiden anderen Gasströme wurde jeweils mit der Temperatur ins Drehrohr geführt, die das Materialgut im Drehrohr jeweils aufwies.
   Ab Überschreiten der Materialguttemperatur von 160°C bis zum Erreichen einer Materialguttemperatur von 300°C wurden aus dem Materialgut 40 Mol-% der im Verlauf der gesamten thermischen Behandlung des Materialgutes insgesamt freigesetzten Ammoniakmenge M^{A} freigesetzt.
- bei Erreichen der Materialguttemperatur von 320°C wurde der Sauerstoffgehalt des dem Drehrohr zugeführten Gasstromes von 0 Vol.-% auf 1,5 Vol.-% erhöht und über die nachfolgenden 4 h beibehalten.
   Gleichzeitig wurde die in den vier das Drehrohr beheizenden Heizzonen herrschende Temperatur um 5°C abgesenkt (auf 325°C) und so während der nachfolgenden 4 h aufrechterhalten.
   Die Materialguttemperatur durchlief dabei ein oberhalb von 325°C liegendes Temperaturmaximum, das 340°C nicht überschritt, bevor die Materialguttemperatur wieder auf 325°C sank.
   Die Zusammensetzung des durch das Drehrohr geführten Gasstroms von 205 Nm³/h wurde während dieses Zeitraums von 4 h wie folgt geändert:
   95 Nm³/h bestehend aus Grundlast - Stickstoff (20) und im Drehrohr freigesetzten Gasen;
   25 Nm³/h Sperrgas - Stickstoff (11);
   70 Nm³/h rezirkuliertes Kreisgas; und
   15 Nm³/h Luft über den Splitter (21).
   Der Sperrgas-Stickstoff wurde mit einer Temperatur von 25°C zugeführt.
   Das Gemisch der anderen Gasströme wurde jeweils mit der Temperatur ins Drehrohr geführt, die das Materialgut im Drehrohr jeweils aufwies.
   Ab Überschreiten der Materialguttemperatur von 300°C bis zum Ablauf der 4 h wurden aus dem Materialgut 55 mol% der im Verlauf der gesamten thermischen Behandlung des Materialguts insgesamt freigesetzten Ammoniakmenge M^{A} freigesetzt (damit waren bis zum Ablauf der 4 h insgesamt 40 Mol-% + 55 Mol-% = 95 Mol-% der Ammoniakmenge M^{A} freigesetzt).
- mit Ablauf der 4 h wurde die Temperatur des Materialguts mit einer Heizrate von 0,85°C/min innerhalb von etwa 1,5 h auf 400°C erhöht.
   Anschließend wurde diese Temperatur 30 min aufrechtgehalten.
   Die Zusammensetzung des dem Drehrohr zugeführten Gasstroms von 205 Nm³/h war während dieser Zeit wie folgt:
   95 Nm³/h zusammengesetzt aus Grundlast - Stickstoff (20) und im Drehrohr freigesetzten Gasen;
   15 Nm³/h Luft (Splitter (21));
   25 Nm³/h Sperrgas-Stickstoff (11); und
   70 Nm³/h rezirkuliertes Kreisgas.
   Der Sperrgas-Stickstoff wurde mit einer Temperatur von 25°C zugeführt. Das Gemisch der anderen Gasströme wurde jeweils mit der Temperatur ins Drehrohr geführt, die das Materialgut im Drehrohr jeweils aufwies.
- durch Verringerung der Temperatur des Materialguts wurde die Calcination beendet; dazu wurden die Heizzonen aus- und die Schnellkühlung des Drehrohres durch Ansaugen von Luft eingeschaltet, und die Materialguttemperatur innerhalb von 2 h auf eine unterhalb von 100°C liegende Temperatur erniedrigt und schließlich auf Umgebungstemperatur abgekühlt;
   mit der Abschaltung der Heizzonen wurde die Zusammensetzung des dem Drehrohr zugeführten Gasstroms von 205 Nm³/h auf folgendes Gemisch verändert:
   110 Nm³/h zusammengesetzt aus Grundlast - Stickstoff (20) und im Drehrohr freigesetzten Gasen;
   0 Nm³/h Luft (Splitter (21));
   25 Nm³/h Sperrgas-Stickstoff (11); und
   70 Nm³/h rezirkuliertes Kreisgas.
   Der Gasstrom wurde dem Drehrohr mit einer Temperatur von 25°C zugeführt.
- während der gesamten thermischen Behandlung lag der Druck (unmittelbar) hinter dem Drehrohrausgang 0,2 mbar unterhalb des Außendrucks.

### 6. Formgebung der Multimetalloxidaktivmasse

Das in "5." erhaltene katalytisch aktive Material wurde mittels einer Biplexquerstromsichtmühle (BQ 500) (Fa. Hosokawa-Alpine Augsburg) zu einem feinteiligen Pulver gemahlen, von dem 50 % der Pulverpartikel ein Sieb der Maschenweite 1 bis 10 µm passierten und dessen Anteil an Partikeln mit einer Längstausdehnung oberhalb von 50 µm weniger als 1 % betrug.

Mittels des gemahlenen Pulvers wurden wie in S1 der EP-B 714700 ringförmige Trägerkörper (7 mm Außendurchmesser, 3 mm Länge, 4 mm Innendurchmesser, Steatit des Typs C220 der Fa. CeramTec mit einer Oberflächenrauhigkeit Rz von 45 µm) beschichtet. Bindemittel war eine wässrige Lösung aus 75 Gew.-% Wasser und 25 Gew.-% Glycerin.

Der Aktivmassenanteil der resultierenden Schalenkatalysatoren wurde jedoch im Unterschied zu vorgenanntem Beispiel S1 zu 20 Gew.-% (bezogen auf das Gesamtgewicht aus Trägerkörper und Aktivmasse) gewählt. Das Verhältnis von Pulver und Bindemittel wurde proportional angepasst.

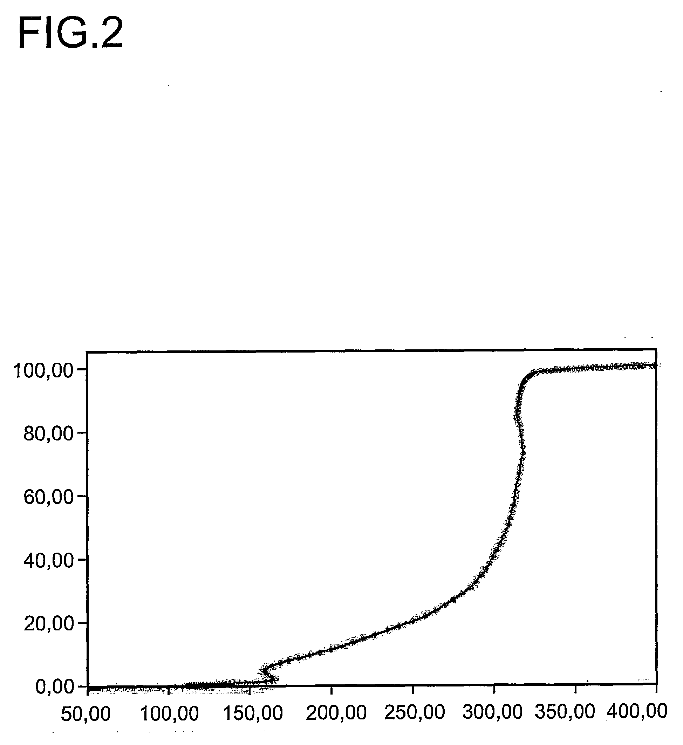

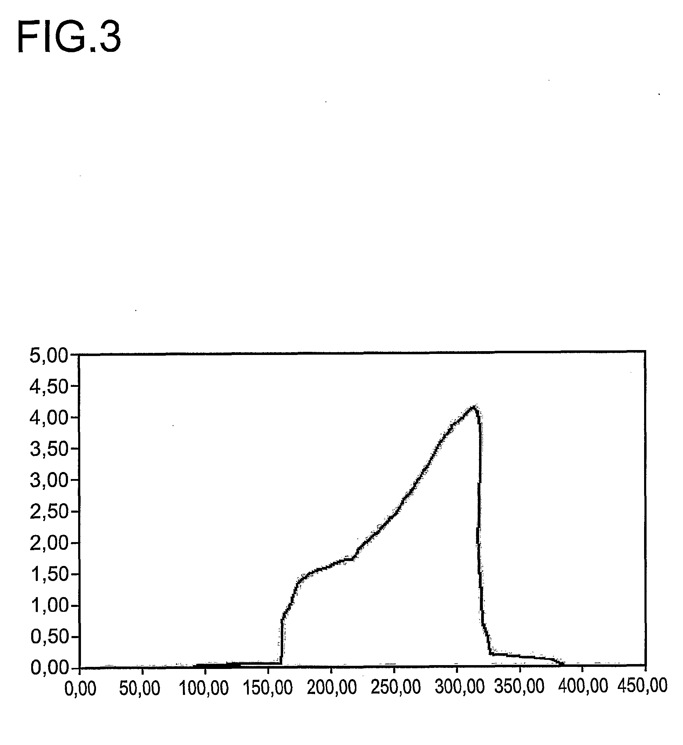

Figur 2 zeigt den prozentualen Anteil von M^{A} als Funktion der Materialguttemperatur in °C. Figur 3 zeigt die Ammoniakkonzentration der Atmosphäre A in Vol.-% über die thermische Behandlung in Abhängigkeit von der Materialguttemperatur in °C.

### C) Durchführung der Partialoxidation

### I. Beschreibung der allgemeinen Verfahrensbedingungen in der ersten Reaktionsstufe

- Verwendetes Wärmeaustauschmittel:: Salzschmelze, bestehend aus 60 Gew.-% Kaliumnitrat und 40 Gew.-% Natriumnitrit.
- Material der Kontaktrohre:: ferritischer Stahl.
- Abmessungen der Kontaktrohre:: 3200 mm Länge;
25 mm Innendurchmesser;
30 mm Außendurchmesser (Wandstärke:
2,5 mm).
- Anzahl der Kontaktrohre im Rohrbündel:: 25500.
- Reaktor:: Zylinderförmiger Behälter eines Durchmessers von 6800 mm; ringförmig angeordnetes Rohrbündel mit einem freien zentralen Raum.
Durchmesser des zentralen freien Raumes: 1000 mm. Abstand der am weitesten außen liegenden Kontaktrohre zur Behälterwand: 150 mm. Homogene Kontaktrohrverteilung im Rohrbündel (6 äquidistante Nachbarrohre pro Kontaktrohr).
Kontaktrohrteilung: 38 mm.
Die Kontaktrohre waren mit ihren Enden in Kontaktrohrböden der Dicke 125 mm abdichtend befestigt und mündeten mit ihren Öffnungen in je eine am oberen bzw. unteren Ende mit dem Behälter verbundenen Haube.
Zuführung des Wärmeaustauschmittels zum Rohrbündel:
Das Rohrbündel war durch drei zwischen den Kontaktrohrböden längs derselben aufeinanderfolgend angebrachte Umlenkscheiben (Dicke jeweils 10 mm) in 4 äquidistante (jeweils 730 mm) Längsabschnitte (Zonen) geteilt.

Die unterste und die oberste Umlenkscheibe wies Ringgeometrie auf, wobei der Ringinnendurchmesser 1000 mm betrug und der Ringaußendurchmesser sich bis zur Behälterwand abdichtend erstreckte. Die Kontaktrohre waren an den Umlenkscheiben nicht abdichtend befestigt. Vielmehr waren eine Spaltbreite < 0,5 mm aufweisende Spalte so belassen, daß die Querströmungsgeschwindigkeit der Salzschmelze innerhalb einer Zone möglichst konstant war.

Die mittlere Umlenkscheibe war kreisförmig und erstreckte sich bis zu den am weitesten außen liegenden Kontaktrohren des Rohrbündels.

Die Kreisführung der Salzschmelze wurde durch zwei Salzpumpen bewerkstelligt, von denen jede eine Rohrbündellängshälfte versorgte.

Die Pumpen drückten die Salzschmelze in einen um den Reaktormantel unten angebrachten Ringkanal, der die Salzschmelze über den Behälterumfang verteilte. Durch im Reaktormantel befindliche Fenster gelangte die Salzschmelze im untersten Längsabschnitt zum Rohrbündel. Die Salzschmelze floß dann der Vorgabe der Umlenkbleche folgend in der Abfolge
- von außen nach innen,
- von innen nach außen,
- von außen nach innen,
- von innen nach außen,
im wesentlichen mäanderförmig, über den Behälter betrachtet, von unten nach oben. Durch im obersten Längsabschnitt um den Behälterumfang angebrachte Fenster sammelte sich die Salzschmelze in einem oberen um den Reaktormantel angebrachten Ringkanal und wurde nach Abkühlung auf die ursprüngliche Eintrittstemperatur durch die Pumpen wieder in den unteren Ringkanal gedrückt.

Die Zusammensetzung des Reaktionsgasausgangsgemisches 1 (Gemisch aus Luft, chemical grade Propylen und Kreisgas) lag über die Betriebszeit in folgendem Raster:
5 bis 7 Vol.-% Propen (chemical grade),
10 bis 14 Vol.-% Sauerstoff,
1 bis 2 Vol.-% COₓ,
1 bis 3 Vol.-% H₂O, und
wenigstens 80 Vol.-% N₂.

| | |
|---|---|
| Reaktorbeschickung: | Salzschmelze und Reaktionsgasgemisch wurden über den Reaktor betrachtet im Gegenstrom geführt. Die Salzschmelze trat unten ein, das Reaktionsgasgemisch oben. |
| | Die Eintrittstemperatur der Salzschmelze betrug am Anfang (nach beendeter Formierung des Katalysatorfestbetts 1) 337°C. |
| | Die Austrittstemperatur der Salzschmelze lag am Anfang bei 339°C. |
| | Die Pumpleistung betrug 6200 m³ Salzschmelze/h. Das Reaktionsgasausgangsgemisch wurde dem Reaktor mit einer Temperatur von 300°C zugeführt. |
| Propenlast des Katalysatorfestbetts 1: | 90 bis 120 Nl/l•h |
| Kontaktrohrbeschickung mit Katalysatorfestbett 1 (von oben nach unten): | Zone A: 50 cm Vorschüttung aus Steatit-Ringen der Geometrie 7 mm x 7 mm x 4 mm (Außendurchmesser x Länge x Innendurchmesser) |
| | Zone B: 100 cm Katalysatorbeschickung mit einem homogenen Gemisch aus 30 Gew.-% an Steatit-Ringen der Geometrie 5 mm x 3 mm x 2 mm (Außendurchmesser x Länge x Innendurchmesser) und 70 Gew.-% des für die erste Reaktionsstufe hergestellten ringförmigen Vollkatalysator). |
| | Zone C: 170 cm Katalysatorbeschickung mit dem für die erste Reaktionsstufe hergestellten ringförmigen (5 mm x 3 mm x 2 mm = Außendurchmesser x Länge x Innendurchmesser) Vollkatalysator. |

Die Thermorohre (ihre Anzahl belief sich auf 10, die im zentralen Bereich des Rohrbündels gleichmäßig verteilt waren) waren wie folgt gestaltet und beschickt: (mit ihnen wurde die Heißpunkttemperatur ermittelt; sie ist ein arithmetischer Mittelwert aus voneinander unabhängigen Messungen in den 10 Thermorohren)
Jedes der 10 Thermorohre wies eine zentrale Thermohülse mit 40 Temperaturmessstellen auf (d.h., jedes Thermorohr enthielt 40 Thermoelemente, die mit unterschiedlicher Länge in eine Thermohülse integriert waren und so ein Multithermoelement bildeten, mit dem innerhalb des Thermorohres auf unterschiedlichen Höhen simultan die Temperatur ermittelt werden konnte).
Wenigstens 13 und höchstens 30 der jeweils 40 Temperaturmessstellen befand sich im Bereich des ersten Meters des aktiven Abschnitts des Katalysatorfestbetts (in Strömungsrichtung des Reaktionsgasgemischs).
Der Innendurchmesser eines Thermorohres betrug 27 mm. Die Wanddicke und das Rohrmaterial war wie bei den Arbeitsrohren beschaffen.
Der Außendurchmesser der Thermohülse betrug 4 mm.
Die Befüllung der Thermorohre erfolgte wie folgt:
Ein Thermorohr wurde mit dem hergestellten ringförmigen Vollkatalysator befüllt. Zusätzlich wurde in das Thermorohr aus dem ringförmigen Vollkatalysator erzeugter Katalysatorsplit der Längstausdehnung 0,5 bis 5 mm beigefüllt.
Die Beifüllung des Katalysatorsplits erfolgte über den gesamten aktiven Abschnitt des Katalysatorfestbetts des jeweiligen Thermorohrs homogen verteilt so, dass der Druckverlust des Reaktionsgasgemischs beim Durchgang durch das Thermorohr demjenigen beim Durchgang des Reaktionsgasgemischs durch ein Arbeitsrohr entsprach (bezogen auf den aktiven Abschnitt des Katalysatorfestbetts (d.h., die Inertabschnitte ausgenommen) im Thermorohr waren dazu 5 bis 20 Gew.-% an Katalysatorsplit erforderlich). Gleichzeitig war die jeweilige Gesamtfüllhöhe von Aktiv- und Inertabschnitten in den Arbeits- und Thermorohren gleich bemessen und das Verhältnis von im Rohr enthaltener Gesamtmenge an Aktivmasse zu Wärmeübergangsfläche des Rohres bei Arbeits- und Thermorohren auf den im wesentlichen selben Wert eingestellt.

### II. Beschreibung der Zwischenkühlung

Das die erste Reaktionsstufe mit einer der Salzschmelzeaustrittstemperatur entsprechenden Temperatur verlassende Produktgasgemisch wurde zum Zweck der Zwischenkühlung durch einen mit einer Salzschmelze aus 60 Gew.-% Kaliumnitrat und 40 Gew.-% Natriumnitrit gekühlten Einzonen-Rohrbündelwärmetauscher aus ferritische Stahl geführt, der unmittelbar an den Reaktor angeflanscht war. Der Abstand des unteren Rohrbodens des Reaktors zum oberen Rohrboden des Kühlers betrug dabei 10 cm. Die Salzschmelze und das Produktgasgemisch wurden dabei über den Wärmetauscher betrachtet im Gegenstrom geführt. Das Salzbad selbst floß in gleicher Weise wie im Erststufen-Einzonen-Vielkontaktrohr-Festbettreaktor mäanderförmig um die Kühlrohre, durch die das Produktgasgemisch geleitet wurde. Die Länge der Kühlrohre betrug 1,65 m, ihr Innendurchmesser lag bei 2,6 cm und ihre Wanddicke war 2,5 mm.

Die Anzahl der Kühlrohre belief sich auf 8000. Der Durchmesser des Wärmetauschers betrug 7,2 m.

Sie waren mit einheitlicher Rohrteilung gleichmäßig über den Querschnitt verteilt.

In den Eingang der Kühlrohre (in Strömungsrichtung) waren Spiralen aus Edelstahl eingeführt, deren Querschnitt nahezu dem der Kühlrohre entsprach. Ihre Länge betrug 700 mm bis 1000 mm (alternativ können die Kühlrohre mit großen Inertmaterialringen befüllt werden). Sie dienten der Verbesserung des Wärmeübergangs.

Das Produktgasgemisch verließ den Zwischenkühler mit einer Temperatur von 250°C. Anschließend wurde ihm komprimierte Luft, die eine Temperatur von 140°C aufwies, in einer Menge etwa 6700 Nm³/h zugemischt, so dass die nachfolgend beschriebene Zusammensetzung des Beschickungsgasgemischs für die zweite Reaktionsstufe resultierte.

Das dabei erhaltene Beschickungsgasgemisch (Reaktionsgasausgangsgemisch 2) wurde mit einer Temperatur von 220°C dem Einzonen-Vielkontaktrohr-Festbettreaktor der zweiten Reaktionsstufe zugeführt.

### III. Beschreibung der allgemeinen Verfahrensbedingungen in der zweiten Reaktionsstufe

Es wurde ein Einzonen-Vielkontaktrohr-Festbettreaktor verwendet, der mit jenem der ersten Stufe baugleich war.

Die Zusammensetzung des Beschickungsgasgemisches (Reaktionsgasausgangsgemischs 2) lag über die Betriebszeit in folgendem Raster:
4 bis 6 Vol.-% Acrolein,
5 bis 8 Vol.-% O₂,
1,2 bis 2,5 Vol.-% COₓ,
6 bis 10 Vol.-% H₂O und
wenigstens 75 Vol.-% N₂.

| | |
|---|---|
| Reaktorbeschickung: | Salzschmelze und Beschickungsgasgemisch wurden über den Reaktor betrachtet im Gegenstrom geführt. Die Salzschmelze trat unten ein, das Beschickungsgasgemisch oben. |
| | Die Eintrittstemperatur der Salzschmelze betrug am Anfang (nach beendeter Formierung des Katalysatorfestbetts 2) ca. 263°C. Die zugehörige Austrittstemperatur der Salzschmelze lag am Anfang bei ca. 265°C. |
| | Die Pumpleistung betrug 6200 m³ Salzschmelze/h. |
| | Das Reaktionsgasausgangsgemisch wurde dem Reaktor mit einer Temperatur von 240°C zugeführt. |
| Acroleinlast des Katalysatorfestbetts 2: | 95 bis 110 Nl/l•h |
| Die Kontaktrohrbeschickung mit Katalysatorfestbett 2 (von oben nach unten) war: | |
| | Zone A: 20 cm Verschüttung aus Steatit-Ringen der Geometrie 7 mm x 7 mm x 4 mm (Außendurchmesser x Länge x Innendurchmesser). |
| | Zone B: 100 cm Katalysatorbeschickung mit einem homogenen Gemisch aus 25 Gew.-% an Steatit-Ringen der Geometrie 7 mm x 3 mm x 4 mm (Außendurchmesser x Länge x Innendurchmesser) und 75 Gew.-% des ringförmigen (ca. 7 mm x 3 mm x 4 mm) für die zweite Reaktionsstufe hergestellten Schalenkatalysators. |
| | Zone C: 200 cm Katalysatorbeschickung mit dem ringförmigen (ca. 7 mm x 3 mm x 4 mm) für die zweite Reaktionsstufe hergestellten Schalenkatalysator. |

Die Thermorohre (ihre Anzahl belief sich auf 10, die im zentralen Bereich des Rohrbündels gleichmäßig verteilt waren) waren wie folgt gestaltet und beschickt: (mit ihnen wurde die Heißpunkttemperatur ermittelt; sie ist ein arithmetischer Mittelwert aus voneinander unabhängigen Messungen in den 10 Thermorohren)
Jeder der 10 Thermorohre wies eine zentrale Thermohülse mit 40 Temperaturmessstellen auf (d.h., jedes Thermorohr enthielt 40 Thermoelemente, die mit unterschiedlicher Länge in eine Thermohülse integriert waren und so ein Multithermoelement bildeten, mit dem innerhalb des Thermorohres auf unterschiedlichen Höhen simultan die Temperatur ermittelt werden konnte).
Wenigstens 13 und höchstens 30 der jeweils 40 Temperaturmessstellen befand sich im Bereich des ersten Meters des aktiven Abschnitts des Katalysatorfestbetts (in Strömungsrichtung des Reaktionsgasgemischs).
Der Innendurchmesser eines Thermorohres betrug 27 mm. Die Wanddicke und das Rohrmaterial war wie bei den Arbeitsrohren beschaffen.
Der Außendurchmesser der Thermohülse betrug 4 mm.
Die Befüllung der Thermorohre erfolgte wie folgt:
   Ein Thermorohr wurde mit dem hergestellten ringförmigen Schalenkatalysator befüllt. Zusätzlich wurde in das Thermorohr kugelförmiger Schalenkatalysator beigefüllt (gleiche Aktivmasse wie der ringförmige Schalenkatalysator, der Durchmesser der Steatit C220 (CeramTec) Trägerkugeln betrug 2-3 mm; der Aktivmassenanteil betrug 20 Gew.-%, die Herstellung erfolgte wie bei dem ringförmigen Schalenkatalysator beschrieben, Bindemittel war jedoch eine entsprechende Menge Wasser).
   Die Beifüllung des kugelförmigen Schalenkatalysators erfolgte über den gesamten aktiven Abschnitt des Katalysatorfestbetts des jeweiligen Thermorohrs homogen verteilt so, dass der Druckverlust des Reaktionsgasgemischs beim Durchgang durch das Thermorohr demjenigen beim Durchgang des Reaktionsgasgemischs durch ein Arbeitsrohr entsprach (bezogen auf den aktiven Abschnitt des Katalysatorfestbetts (d.h., die Inertabschnitte ausgenommen) im Thermorohr waren dazu 5 bis 20 Gew.-% an kugelförmigem Schalenkatalysator erforderlich). Gleichzeitig war die jeweilige Gesamtfüllhöhe von Aktiv- und Inertabschnitten in den Arbeits- und Thermorohren gleich bemessen und das Verhältnis von im Rohr enthaltener Gesamtmenge an Aktivmasse zu Wärmeübergangsfläche des Rohres bei Arbeits- und Thermorohren auf denselben Wert eingestellt.

### III. Langzeitbetrieb (Ergebnisse)

Das Umsatzziel für das bei einmaligem Durchgang des Reaktionsgasgemischs 1 durch das Katalysatorfestbett 1 umzusetzende Propen wurde auf 97,5 mol-% festgelegt.

Durch sukzessives Erhöhen der Eintrittstemperatur der Salzschmelze in den Erststufenreaktor konnte dieser Umsatzwert bei kontinuierlicher Durchführung des Verfahrens über die Zeit aufrechterhalten werden.

Das Umsatzziel für das bei einmaligem Durchgang des Reaktionsgasausgangsgemisches 2 durch das Katalysatorfestbett 2 umzusetzende Acrolein wurde auf 99,3 mol-% festgelegt.

Durch sukzessives Erhöhen der Eintrittstemperatur der Salzschmelze in den Zweitstufenreaktor konnte dieser Umsatzwert bei kontinuierlicher Durchführung des Verfahrens über die Zeit aufrechterhalten werden.

Einmal je Kalendermonat wurde die Partialoxidation unterbrochen (die Erhöhung der Eintrittstemperatur der Salzschmelze in den Zweitstufenreaktor bis zur monatlichen Unterbrechung betrug stets ≥ 0,3°C und ≤ 4°C; im Fall des Erststufenreaktors lagen die monatlich erforderlichen Erhöhungswerte bei ≥ 0,5°C), die in der jeweiligen Reaktionsstufe sowie im Zwischenkühler zuletzt angewandte Eintrittstemperatur der Salzschmelze beibehalten und für eine Zeitdauer t_{G} von 24 h bis 48 h ein Gasgemisch G aus 6 Vol.-% O₂ und 95 Vol.-% N₂ mit einer Belastung des Katalysatorfestbetts 1 von 30 Nl/l·h durch das gesamte Reaktorsystem geführt.

Danach wurde die Partialoxidation fortgesetzt und die Eintrittstemperaturen der Salzschmelze in die jeweilige Reaktionsstufe so eingestellt, dass das Umsatzziel der jeweiligen Reaktionsstufe weiter erreicht wurde.

Die Eintrittstemperaturen der Salzschmelzen und die Heißpunkttemperaturen sowie die Selektivität S^{AC+AA} der Summe aus Acroleinbildung und Acrylsäurenebenproduktbildung in der ersten Reaktionsstufe und die Selektivität S^{AA} der Acrylsäurebildung in der zweiten Reaktionsstufe (jeweils bezogen auf umgesetztes Propen) entwickelten sich dabei wie folgt:

**Erste Reaktionsstufe:**

| | | |
|---|---|---|
| Anfang: | Salzschmelzeeintrittstemperatur = 316°C | |
| | Heißpunkttemperatur = 366°C | |
| | S^{AC+AA} = 94,5 mol-% | |
| | | |
| nach 1 Jahr Betriebsdauer: | Salzschmelzeeintrittstemperatur = 319°C | |
| | Heißpunkttemperatur = 370°C | |
| | S^{AC+AA} = 94,8 mol-% | |
| | | |
| nach 2 Jahren Betriebsdauer: | Salzschmelzeeintrittstemperatur = 324°C | |
| | Heißpunkttemperatur = 371°C | |
| | S^{AC+AA} = 95,1 mol-% | |
| | | |
| nach 3 Jahren Betriebsdauer: | Salzschmelzeeintrittstemperatur = 330°C | |
| | Heißpunkttemperatur = 376°C | |
| | S^{AC+AA} = 95,4 mol-% | |
| | | |
| Zweite Reaktionsstufe: | | |
| | | |
| Anfang: | Salzschmelzeeintrittstemperatur | = 263°C |
| | Heißpunkttemperatur | = 290°C |
| | S^{AA} | = 89,2 mol-% |
| | | |
| Nach 1 Jahr | | |
| Betriebsdauer: | Salzschmelzeeintrittstemperatur | = 268°C |
| | Heißpunkttemperatur | = 292°C |
| | S^{AA} | = 89,4 mol-% |
| | | |
| Nach 2 Jahren | | |
| Betriebsdauer: | Salzschmelzeeintrittstemperatur | = 274°C |
| | Heißpunkttemperatur | = 297°C |
| | S^{AA} | = 89,7 mol-% |
| | | |
| Nach 3 Jahren | | |
| Betriebsdauer: | Salzschmelzeeintrittstemperatur | = 278°C |
| | Heißpunkttemperatur | = 300°C |
| | S^{AA} | = 90,2 mol-% |

Die Temperaturangaben (abgesehen für den Anfang) beziehen sich in allen Fällen jeweils auf den Zeitpunkt, der kurz vor der Unterbrechung der Partialoxidation und der Behandlung der Katalysatorbetten mit dem Gasgemisch G lag.

Innerhalb der 3 Jahre Betriebsdauer wanderte der Ort der Heißpunkttemperatur im Katalysatorfestbett 2 etwa um ca. 25 cm in Richtung der Eintrittsstelle des Beschickungsgasgemischs.

Das Beispiel kann in entsprechender Weise auch mit einem Reaktionsgasausgangsgemisch 1 für die erste Reaktionsstufe mit der nachfolgenden Zusammensetzung durchgeführt werden:
6,0 Vol.-% Propen,
60 Vol.-% Luft und
34 Vol.-% H₂O.

Alternativ können für die erste Reaktionsstufe auch Reaktionsgasausgangsgemische 1 der Zusammensetzung gemäß Example 1 der EP-A 990 636, oder gemäß Example 2 der EP-A 960 636 oder gemäß Example 3 der EP-A 1 106 598, oder gemäß Example 26 der EP-A 1 106 598, oder gemäß Example 53 der EP-A 1 106 598 verwendet werden. Sekundärlufteinspeisung erfolgt dabei nur in dem Umfang, dass im Beschickungsgas für die zweite Reaktionsstufe (Reaktionsgasausgangsgemisch 2) O₂:C₃H₄O ≥ 0,5 und ≤ 1,5 erfüllt ist.

US Provisional Patent Application No. 60/515,659, eingereicht am 31. Oktober 2003 ist eingefügt in die vorliegende Anmeldung durch Literaturhinweis.

Im Hinblick auf die obengenannten Lehren sind zahlreiche Änderungen und Abweichung von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

## Patentansprüche

1. Ein Verfahren zum Langzeitbetrieb einer heterogen katalysierten Gasphasenpartialoxidation von Propen zu Acrylsäure, bei dem man ein Propen, molekularen Sauerstoff und wenigstens ein Inertgas enthaltendes Reaktionsgasausgangsgemisch 1, das den molekularen Sauerstoff und das Propen in einem molaren Verhältnis O₂:C₃H₆ ≥ 1 enthält, zunächst in einer ersten Reaktionsstufe bei erhöhter Temperatur so über ein erstes Katalysatorfestbett 1, dessen Katalysatoren so beschaffen sind, dass ihre Aktivmasse wenigsten ein Molybdän und/oder Wolfram sowie wenigstens eines der Elemente Wismut, Tellur, Antimon, Zinn und Kupfer enthaltendes Multimetalloxid ist, führt, dass der Propenumsatz bei einmaligem Durchgang ≥ 93 mol-% und die damit einhergehende Selektivität der Acroleinbildung sowie der Acrylsäurenebenproduktbildung zusammengenommen ≥ 90 mol-% betragen, die Temperatur des die erste Reaktionsstufe verlassenden Produktgasgemischs 1 durch direkte und/oder indirekte Kühlung gegebenenfalls verringert und dem Produktgasgemisch 1 gegebenenfalls molekularen Sauerstoff und/oder Inertgas zugibt, und danach das Produktgasgemisch 1 als Acrolein, molekularen Sauerstoff und wenigstens ein Inertgas enthaltendes Reaktionsgasausgangsgemisch 2, das den molekularen Sauerstoff und das Acrolein in einem molaren Verhältnis O₂:C₃H₄O ≥ 0,5 enthält, in einer zweiten Reaktionsstufe bei erhöhter Temperatur so über ein zweites Katalysatorfestbett 2, dessen Katalysatoren so beschaffen sind, dass ihre Aktivmasse wenigstens ein die Elemente Molybdän und Vanadin enthaltendes Multimetalloxid ist, führt, dass der Acroleinumsatz bei einmaligem Durchgang ≥ 90 mol-% und die Selektivität der über beide Reaktionsstufen bilanzierten Acrylsäurebildung, bezogen auf umgesetztes Propen, ≥ 80 mol-% beträgt, und bei dem man, um der Deaktivierung sowohl des Katalysatorfestbetts 1 als auch des Katalysatorfestbetts 2 entgegenzuwirken, die Temperatur des jeweiligen Katalysatorfestbetts unabhängig voneinander über die Zeit erhöht, **dadurch gekennzeichnet,**
**dass** man die Gasphasenpartialoxidation wenigstens einmal innerhalb von 7500 Betriebsstunden unterbricht, und bei einer Temperatur des Katalysatorfestbetts 1 von 250 bis 550°C und einer Temperatur des Katalysatorfestbetts 2 von 200 bis 450°C ein aus molekularem Sauerstoff, Inertgas und gegebenenfalls Wasserdampf bestehendes Gasgemisch G zunächst durch das Katalysatorfestbett 1, daran anschließend gegebenenfalls durch einen Zwischenkühler und abschließend durch das Katalysatorfestbett 2 führt.

2. Verfahren nach Anspruch 1, **dadurch gekennzeichnet, dass** man, die Gasphasenpartialoxidation wenigstens einmal innerhalb von 4000 Betriebsstunden unterbricht und das Gasgemisch G durch die Katalysatorbetten führt.

3. Verfahren nach Anspruch 1 oder 2, **dadurch gekennzeichnet, dass** die Zeitdauer während der das Gasgemisch G durch die Katalysatorfestbetten geführt wird, 2h bis 120h beträgt.

4. Verfahren nach einem der Ansprüche 1 bis 3, **dadurch gekennzeichnet, dass** das Gasgemisch G, das durch die Katalysatorfestbetten geführt wird, wenigstens 4 Vol.-% Sauerstoff enthält.

5. Verfahren nach einem der Ansprüche 1 bis 4, **dadurch gekennzeichnet, dass** die Aktivmasse der Katalysatoren des Katalysatorfestbetts 1 wenigstens ein Multimetalloxid der allgemeinen Formel I,
Mo₁₂BiₐFe_{b}X¹_{c}X²_{d}X³ₑX⁴_{f}Oₙ (I),
in der die Variablen nachfolgende Bedeutung aufweisen:
X¹ = Nickel und/oder Kobalt,
X²= Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,
a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,
ist.

6. Verfahren nach einem der Ansprüche 1 bis 5, **dadurch gekennzeichnet, dass** die Aktivmasse der Katalysatoren des Katalysatorfestbetts 2 wenigstens ein Multimetalloxid der allgemeinen Formel IV,
Mo₁₂VₐX¹_{b}X²_{c}X³_{d}X⁴ₑX⁵_{f}X⁶_{g}Oₙ (IV),
in der die Variablen folgende Bedeutung haben:
X¹ = W, Nb, Ta, Cr und/oder Ce,
X² = Cu, Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, Al, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in IV bestimmt wird,
ist.

7. Verfahren nach einem der Ansprüche 1 bis 6, **dadurch gekennzeichnet, dass** die Propenbelastung des Katalysatorfestbetts 1 ≥ 90 Nl/l•h beträgt.

8. Verfahren nach einem der Ansprüche 1 bis 6, **dadurch gekennzeichnet, dass** die Propenbelastung des Katalysatorfestbetts 1 ≥ 130 Nl/l•h beträgt.

9. Verfahren nach einem der Ansprüche 1 bis 8, **dadurch gekennzeichnet, dass** der Propengehalt im Reaktionsgasausgangsgemisch 1 7 bis 15 Vol.-% beträgt.

10. Verfahren nach einem der Ansprüche 1 bis 9, **dadurch gekennzeichnet, dass** der Propengehalt im Reaktionsgasausgangsgemisch 1 8 bis 12 Vol.-% beträgt.

11. Verfahren nach einem der Ansprüche 1 bis 10, **dadurch gekennzeichnet, dass** der Acroleingehalt im Reaktionsgasausgangsgemisch 2 6 bis 15 Von.-% beträgt.

12. Verfahren nach einem der Ansprüche 1 bis 11, **dadurch gekennzeichnet, dass** die Erhöhung der Temperatur des Katalysatorfestbetts 1 über die Zeit so durchgeführt wird, dass der Propengehalt im Produktgasgemisch 1 der ersten Reaktionsstufe 10000 Gew.-ppm nicht übersteigt.

13. Verfahren nach einem der Ansprüche 1 bis 11, **dadurch gekennzeichnet, dass** die Erhöhung der Temperatur des Katalysatorfestbetts 1 über die Zeit so durchgeführt wird, dass der Umsatz des Propens bei einmaligem Durchgang des Reaktionsgasgemischs 1 durch das Katalysatorfestbett 1 94 mol-% nicht unterschreitet.

14. Verfahren nach einem der Ansprüche 1 bis 13, **dadurch gekennzeichnet, dass** die Erhöhung der Temperatur des Katalysatorfestbetts 2 über die Zeit so durchgeführt wird, dass der Acroleingehalt im Produktgasgemisch der zweiten Reaktionsstufe 1500 Gew.-ppm nicht übersteigt.

15. Verfahren nach einem der Ansprüche 1 bis 13, **dadurch gekennzeichnet, dass** die Erhöhung der Temperatur des Katalysatorfestbetts 2 über die Zeit so durchgeführt wird, dass der Umsatz des Acroleins bei einmaligem Durchgang des Reaktionsgasgemischs durch das Katalysatorfestbett 2 92 mol-% nicht unterschreitet.

16. Verfahren nach einem der Ansprüche 1 bis 15, **dadurch gekennzeichnet, dass** beide Reaktionsstufen in einem Rohrbündelreaktor durchgeführt werden.

## Claims

1. A process for the long-term operation of a heterogeneously catalyzed gas phase partial oxidation of propene to acrylic acid, by initially conducting a starting reaction gas mixture 1 which comprises propene, molecular oxygen and at least one inert gas and contains the molecular oxygen and the propene in a molar O₂:C₃H₆ ratio of ≥ 1, in a first reaction stage, at elevated temperature, over a first fixed catalyst bed 1 whose catalysts are such that their active composition is at least one multimetal oxide containing molybdenum and/or tungsten and also at least one of the elements bismuth, tellurium, antimony, tin and copper, in such a way that the propene conversion in single pass is ≥ 93 mol% and the accompanying selectivity of acrolein formation and of acrylic acid by-production taken together are ≥ 90 mol%, optionally reducing the temperature of the product gas mixture 1 leaving the first reaction stage by direct and/or indirect cooling and optionally adding molecular oxygen and/or inert gas to the product gas mixture 1, and then conducting the product gas mixture 1, as a starting reaction gas mixture 2 which comprises acrolein, molecular oxygen and at least one inert gas and contains the molecular oxygen and the acrolein in a molar O₂:C₃H₄O ratio of ≥ 0.5, in a second reaction stage, at elevated temperature, over a second fixed catalyst bed 2 whose catalysts are such that their active composition is at least one multimetal oxide containing the elements molybdenum and vanadium, in such a way that the acrolein conversion in single pass is ≥ 90 mol% and the selectivity of acrylic acid formation assessed over both reaction stages, based on propene converted, is ≥ 80 mol%, and by, in order to counteract the deactivation both of the fixed catalyst bed 1 and of the fixed catalyst bed 2, independently increasing the temperature of the particular fixed catalyst bed over time, which comprises
interrupting the gas phase partial oxidation at least once within 7500 operating hours, and, at a temperature of the fixed catalyst bed 1 of from 250 to 550°C and a temperature of the fixed catalyst bed 2 of from 200 to 450°C initially conducting a gas mixture G consisting of molecular oxygen, inert gas and optionally steam through the fixed catalyst bed 1, and subsequently optionally through an intermediate cooler and finally through the fixed catalyst bed 2.

2. The process according to claim 1, wherein the gas phase partial oxidation is interrupted at least once within 4000 operating hours and the gas mixture G is conducted through the catalyst beds.

3. The process according to claim 1 or 2, wherein the period over which the gas mixture G is conducted through the fixed catalyst beds is from 2h to 120h.

4. The process according to any of claims 1 to 3, wherein the gas mixture G which is conducted through the fixed catalyst beds contains at least 4% by volume of oxygen.

5. The process according to any of claims 1 to 4, wherein the active composition of the catalysts of the fixed catalyst bed 1 is at least one multimetal oxide of the general formula I
Mo₁₂BiₐFe_{b}X¹_{c}X²_{d}X³ₑX⁴_{f}Oₙ (I)
in which the variables are each defined as follows:
X¹= nickel and/or cobalt,
X²= thallium, an alkali metal and/or an alkaline earth metal,
X³= zinc, phosphorus, arsenic, boron, antimony, tin, cerium, lead and/or tungsten,
X⁴= silicon, aluminum, titanium and/or zirconium,
a = from 0.5 to 5,
b = from 0.01 to 5, preferably from 2 to 4,
c = from 0 to 10, preferably from 3 to 10,
d = from 0 to 2, preferably from 0.02 to 2,
e = from 0 to 8, preferably from 0 to 5,
f = from 0 to 10 and
n = a number which is determined by the valency and frequency of the elements in I other than oxygen.

6. The process according to any of claims 1 to 5, wherein the active composition of the catalysts of the fixed catalyst bed 2 is at least one multimetal oxide of the general formula IV
Mo₁₂VₐX¹_{b}X²_{c}X³_{d}X⁴ₑX⁵_{f}X⁶_{g}Oₙ (IV)
in which the variables are each defined as follows:
X¹ = W, Nb, Ta, Cr and/or Ce,
X² = Cu, Ni, Co, Fe, Mn and/or Zn,
X³ = Sb and/or Bi,
X⁴ = one or more alkali metals,
X⁵ = one or more alkaline earth metals,
X⁶ = Si, Al, Ti and/or Zr,
a = from 1 to 6,
b = from 0.2 to 4,
c = from 0.5 to 18,
d =from 0 to 40,
e = from 0 to 2,
f = from 0 to 4,
g =from 0 to 40 and
n =a number which is determined by the valency and frequency of the elements in IV other than oxygen.

7. The process according to any of claims 1 to 6, wherein the propene hourly space velocity on the fixed catalyst bed 1 is ≥ 90 1 (STP)/l•h.

8. The process according to any of claims 1 to 6, wherein the propene hourly space velocity on the fixed catalyst bed 1 is ≥ 130 1 (STP)/l•h.

9. The process according to any of claims 1 to 8, wherein the propene content in the starting reaction gas mixture 1 is from 7 to 15% by volume.

10. The process according to any of claims 1 to 9, wherein the propene content in the starting reaction gas mixture 1 is from 8 to 12% by volume.

11. The process according to any of claims 1 to 10, wherein the acrolein content in the starting reaction gas mixture 2 is from 6 to 15% by volume.

12. The process according to any of claims 1 to 11, wherein the increase in the temperature of the fixed catalyst bed 1 over time is carried out in such a way that the propene content in the product gas mixture 1 of the first reaction stage does not exceed 10 000 ppm by weight.

13. The process according to any of claims 1 to 11, wherein the increase in the temperature of the fixed catalyst bed 1 over the time is carried out in such a way that the conversion of propene in single pass of the reaction gas mixture 1 through the fixed catalyst bed 1 does not go below 94 mol%.

14. The process according to any of claims 1 to 13, wherein the increase in the temperature of the fixed catalyst bed 2 over the time is carried out in such a way that the acrolein content in the product gas mixture of the second reaction stage does not exceed 1 500 ppm by weight.

15. The process according to any of claims 1 to 13, wherein the increase in the temperature of the fixed catalyst bed 2 over the time is carried out in such a way that the conversion of acrolein in single pass of the reaction gas mixture through the fixed catalyst bed 2 does not go below 92 mol%.

16. The process according to any of claims 1 to 15, wherein both reaction stages are carried out in a tube bundle reactor.

## Revendications

1. Procédé pour le fonctionnement à long terme d'une oxydation partielle en phase gazeuse catalysée par voie hétérogène de propylène en acide acrylique, dans lequel un mélange 1 initial gazeux réactionnel, contenant du propylène, de l'oxygène moléculaire et au moins un gaz inerte, qui contient l'oxygène moléculaire et le propylène dans un rapport molaire de O₂:C₃H₆ ≥ 1, est d'abord guidé, dans une première étape de réaction à température augmentée, sur un premier lit fixe catalytique 1, dont les catalyseurs sont réalisés de manière telle que leur masse active est au moins un oxyde multimétallique contenant du molybdène et/ou du tungstène ainsi qu'au moins un des éléments bismuth, tellure, antimoine, étain et cuivre, de manière telle que la conversion du propylène lors d'un passage unique est ≥ 93% en mole et la sélectivité allant de pair avec celle-ci de la formation d'acroléine ainsi que de la formation du produit secondaire acide acrylique prises ensemble est ≥ 90% en mole, la température du mélange 1 gazeux produit quittant la première étape de réaction est le cas échéant abaissée par refroidissement direct et/ou indirect et le mélange 1 gazeux produit est le cas échéant additionné d'oxygène moléculaire et/ou de gaz inerte, puis le mélange 1 gazeux produit est guidé, en tant que mélange 2 initial gazeux réactionnel contenant de l'acroléine, de l'oxygène moléculaire et au moins un gaz inerte, qui contient l'oxygène moléculaire et l'acroléine dans un rapport molaire de O₂:C₃H₄O ≥ 0,5, dans une deuxième étape de réaction à température augmentée sur un deuxième lit fixe catalytique 2, dont les catalyseurs sont réalisés de manière telle que leur masse active est au moins un oxyde de multimétallique contenant du molybdène et du vanadium, de manière telle que la conversion de l'acroléine lors d'un passage unique est ≥ 90% en mole et la sélectivité de la formation d'acide acrylique au total sur les deux étapes de réaction, par rapport au propylène transformé, est ≥ 80% en mole, et dans lequel, pour s'opposer à la désactivation tant du lit fixe catalytique 1 que du lit fixe catalytique 2, la température de chaque lit catalytique fixe, indépendamment l'un de l'autre, est augmentée en fonction du temps, **caractérisé en ce qu'**on interrompt l'oxydation partielle en phase gazeuse au moins une fois en 7500 heures de fonctionnement et on fait passer, à une température du lit fixe catalytique 1 de 250 à 550°C et à une température du lit fixe catalytique 2 de 200 à 450°C, un mélange gazeux G constitué par de l'oxygène moléculaire, du gaz inerte et le cas échéant de la vapeur d'eau, d'abord à travers le lit fixe catalytique 1, puis ensuite le cas échéant à travers un refroidisseur intermédiaire et enfin à travers le lit fixe catalytique 2.

2. Procédé selon la revendication 1, **caractérisé en ce qu'**on interrompt l'oxydation partielle en phase gazeuse au moins une fois en 4000 heures de fonctionnement et on fait passer le mélange gazeux G à travers les lits catalytiques.

3. Procédé selon la revendication 1 ou 2, **caractérisé en ce que** la durée pendant laquelle le mélange gazeux G est guidé à travers les lits fixes catalytiques est de 2 h à 120 h.

4. Procédé selon l'une quelconque des revendications 1 à 3, **caractérisé en ce que** le mélange gazeux G, qui est guidé à travers les lits fixes catalytiques, contient au moins 4% en volume d'oxygène.

5. Procédé selon l'une quelconque des revendications 1 à 4, **caractérisé en ce que** la masse active des catalyseurs du lit fixe catalytique 1 contient au moins un oxyde multimétallique de formule générale I,
Mo₁₂BiₐFe_{b}X¹_{c}X²_{d}X³ₑX⁴_{f}Oₙ (I),
dans laquelle les variables ont la signification suivante :
X¹ = nickel et/ou cobalt,
X² = thallium, un métal alcalin et/ou un métal alcalino-terreux,
X³ = zinc, phosphore, arsenic, bore, antimoine, étain, cérium, plomb et/ou tungstène,
X⁴ = silicium, aluminium, titane et/ou zirconium,
a = 0,5 à 5,
b = 0,01 à 5, de préférence 2 à 4,
c = 0 à 10, de préférence 3 à 10,
d = 0 à 2, de préférence 0,02 à 2,
e = 0 à 8, de préférence 0 à 5,
f = 0 à 10 et
n = un nombre qui est déterminé par la valence et la fréquence des éléments différents de l'oxygène dans I.

6. Procédé selon l'une quelconque des revendications 1 à 5, **caractérisé en ce que** la masse active des catalyseurs du lit fixe catalytique 2 contient au moins un oxyde multimétallique de formule générale IV,
Mo₁₂VₐX¹_{b}X²_{c}X³_{d}X⁴ₑX⁵_{f}X⁶_{g}Oₙ (IV),
dans laquelle les variables ont la signification suivante :
X¹ = W, Nb, Ta, Cr et/ou Ce,
X² = Cu, Ni, Co, Fe, Mn et/ou Zn,
X³ = Sb et/ou Bi,
X⁴ = un ou plusieurs métaux alcalins,
X⁵ = un ou plusieurs métaux alcalino-terreux,
X⁶ = Si, Al, Ti et/ou Zr,
a = 1 à 6,
b = 0,2 à 4,
c = 0,5 à 18,
d = 0 à 40,
e = 0 à 2,
f = 0 à 4,
g = 0 à 40 et
n = un nombre qui est déterminé par la valence et la fréquence des éléments différents de l'oxygène dans IV.

7. Procédé selon l'une quelconque des revendications 1 à 6, **caractérisé en ce que** la charge en propylène du lit fixe catalytique 1 est ≥ 90 Nl/l*h.

8. Procédé selon l'une quelconque des revendications 1 à 6, **caractérisé en ce que** la charge en propylène du lit fixe catalytique 1 est ≥ 130 Nl/l*h.

9. Procédé selon l'une quelconque des revendications 1 à 8, **caractérisé en ce que** la teneur en propylène dans le mélange 1 initial gazeux réactionnel est de 7 à 15% en volume.

10. Procédé selon l'une quelconque des revendications 1 à 9, **caractérisé en ce que** la teneur en propylène dans le mélange 1 initial gazeux réactionnel est de 8 à 12% en volume.

11. Procédé selon l'une quelconque des revendications 1 à 10, **caractérisé en ce que** la teneur en acroléine dans le mélange 2 initial gazeux réactionnel est de 6 à 15% en volume.

12. Procédé selon l'une quelconque des revendications 1 à 11, **caractérisé en ce que** l'augmentation de la température du lit fixe catalytique 1 en fonction du temps est réalisée de manière telle que la teneur en propylène dans le mélange 1 gazeux produit de la première étape de réaction ne dépasse pas 10.000 ppm en poids.

13. Procédé selon l'une quelconque des revendications 1 à 11, **caractérisé en ce que** l'augmentation de la température du lit fixe catalytique 1 en fonction du temps est réalisée de manière telle que la conversion du propylène lors d'un passage unique du mélange 1 gazeux réactionnel à travers le lit fixe catalytique 1 ne passe pas sous 94% en mole.

14. Procédé selon l'une quelconque des revendications 1 à 13, **caractérisé en ce que** l'augmentation de la température du lit fixe catalytique 2 en fonction du temps est réalisée de manière telle que la teneur en acroléine dans le mélange gazeux produit de la deuxième étape de réaction ne dépasse pas 1500 ppm en poids.

15. Procédé selon l'une quelconque des revendications 1 à 13, **caractérisé en ce que** l'augmentation de la température du lit fixe catalytique 2 en fonction du temps est réalisée de manière telle que la conversion de l'acroléine lors d'un passage unique du mélange gazeux réactionnel à travers le lit fixe catalytique 2 ne passe pas sous 92% en mole.

16. Procédé selon l'une quelconque des revendications 1 à 15, **caractérisé en ce que** les deux étapes de réaction sont réalisées dans un réacteur à faisceau tubulaire.
